# EUROPEAN PATENT SPECIFICATION

(11) **EP 4 422 516 B1**
(45) Date of publication and mention of the grant of the patent: **02.12.2026**
(21) Application number: 22888287.4
(22) Date of filing: 31.10.2022
(51) Int. Cl.: A61B 17/00, A61M 25/01, A61B 17/34, A61B 17/29, A61B 1/00, A61B 1/005

(54) **AGONIST-ANTAGONIST TUBE STEERABLE INSTRUMENT WITH SERPENTINE BEAM ELEMENTS**
AGONIST-ANTAGONIST ROHRSTEUERBARES INSTRUMENT MIT SCHLANGENFÖRMIGEN STRAHLELEMENTEN
INSTRUMENT ORIENTABLE À TUBE ANTAGONISTE-AGONISTE AVEC DES ÉLÉMENTS FORMANT POUTRE EN SERPENTIN

(30) Priority: 29.10.2021 US 202163273679 P
(43) Date of publication of application: 04.09.2024
(73) Proprietor: Vanderbilt University, Nashville, TN 37240 (US); University of Tennessee Research Foundation, Knoxville, Tennessee 37902 (US)
(72) Inventor: GAFFORD, Joshua, Nashville, TN 37240 (US); RUCKER, Daniel C., Nashville, TN 37240 (US); ANDERSON, Patrick L., Nashville, TN 37240 (US); WEBSTER, Scott J., Nashville, TN 37240 (US); WEBSTER, Robert James, Nashville, TN 37240 (US)
(74) Representative: Marks & Clerk LLP
(86) International application number: PCT/US2022/048408
(87) International publication number: WO 2023/076667

(56) References cited:
- WO-A1-2016/160694
- US-A1- 2013 345 709
- US-A1- 2018 028 199
- US-A1- 2018 099 119
- US-A1- 2018 289 388
- US-A1- 2019 133 705
- US-A1- 2020 146 744

## Description

### Related Application

This application claims priority from U.S. Provisional Application No. 63/273,679, filed 29 October 2021.

### Government Funding

This invention was made with government support under grant numbers R44DK126606, R44DC019894 and R44EB031741 awarded by National Institutes of Health. The government has certain rights in the invention.

### Technical Field

This disclosure relates to steerable medical devices configured to perform surgical procedures. More specifically, this disclosure relates to a steerable medical device constructed with an agonist-antagonist tube steerable instrument that implements serpentine beam elements.

### Background

There is a pressing need in flexible endoscopy to create steerable surgical tools that can provide dexterity at the tip of flexible endoscopes. Steered through the endoscope to the surgical site, these tools are especially important in dexterity-driven tasks, such as tissue retraction and resection in colorectal endoscopy, or access-driven tasks, such as reaching around corners to retrieve difficult-to-reach kidney stones in a flexible ureteroscopy. Controlled either manually or robotically, these trans-endoscopic tools can enable physicians to perform tasks that would otherwise be prohibitively difficult or impossible with the native functions of the endoscope alone.
US2019/133705 A1 discloses a small diameter surgical tool which implements an agonist-antagonist deflectable joint. The deflectable joint is an actuatable bendable structure that uses push-pull, agonist-antagonist action of a pair of nested tubes to actuate the joint. The tubes are designed to have non-central, offset neutral axes, and they are fixed together at locations distal to the deflectable joint, such as at their distal ends. Axial translations of the tubes relative to each other causes a push-pull, agonist-antagonist action between the tubes, which causes the deflectable joint to bend. In one implementation, a deflectable joint can be created in nested tubes by configuring radial portions of the tube sidewalls extending along the joint to have an axial region of reduced stiffness. As a result, axial agonist-antagonist motion between the tubes can cause bending of the deflectable joint.
WO2016/160694 A1 discloses steerable medical devices and methods of use. In some examples the steerable medical devices include a steerable portion with a stiffness that varies along the length of the steerable portion. The medical device may have a first portion comprised of a first material having a first durometer and second portion formed of a second material having a second durometer. The first and second portions may be joined at a seam angled relative to the longitudinal axis of the medical device.

### Summary

A surgical system includes a steerable instrument. A surgical system including a steerable instrument according to the present disclosure is defined by appended claim 1. Further embodiments of the surgical system including a steerable instrument are defined by appended claims 2 to 15. According to one example, the steerable instrument can include a first tube including a first serpentine beam formed in a tubular sidewall thereof, and a second tube including a second serpentine beam formed in a tubular sidewall thereof. The first and second tubes are concentrically nested and positioned so that the first and second serpentine beams are at least partially aligned with each other axially and face in radial directions that differ angularly from one another. The first and second tubes are connected to each other distally of the first and second serpentine beams. The first and second serpentine beams define a bending segment that is actuatable to form a bend in the nested tube structure in response to differential axial forces applied to the first and second tubes.

According to another example, the first tube can include a plurality of parallel first slots that extend through the sidewall of the first tube perpendicular to a central axis of the first tube. The first slots are offset angularly in a back-and-forth manner. The first slots define the first serpentine beam. The second tube can include a plurality of parallel second slots that extend through the sidewall of the second tube perpendicular to a central axis of the second tube. The second slots are offset angularly in a back-and-forth manner. The second slots define the second serpentine beam.

According to another example, the first serpentine beam can include lateral beam portions that extend parallel to each other on opposite sides of the first slots, and longitudinal beam portions that interconnect adjacent ends of the lateral beam portions in an alternating manner. The second serpentine beam can include lateral beam portions that extend parallel to each other on opposite sides of the second slots, and longitudinal beam portions that interconnect adjacent ends of the lateral beam portions in an alternating manner.

According to another example, the first and second serpentine beams can be configured so that adjacent lateral beam portions of the first and second serpentine beams engage each other at touch points located opposite longitudinal beam portions connecting the lateral beam portions, wherein the touch points limit deflection of the bending segment.

According to another example, the first and second serpentine beams can be configured so that adjacent lateral beam portions of the first and second serpentine beams engage each other at touch points that limit deflection of the bending segment.

According to another example, the touch points can be configured to define a fully deflected condition of the bending segment.

According to another example, the bending segment can be configured so that the differential axial forces applied to the first and second tubes urge the touch points against each other to increase the stiffness of the bending segment in the fully deflected condition.

According to another example, the first tube can include a plurality of first serpentine beams that extend parallel to each other along the length of the bending segment and the second tube can include a plurality of second serpentine beams that extend parallel to each other along the length of the bending segment.

According to another example, the first tube can include a plurality of parallel first slots that extend through the sidewall of the first tube perpendicular to a central axis of the first tube. The first slots can be arranged in rows of plural slots spaced radially from each other. Each row of first slots can be offset angularly from adjacent rows, wherein the first slots define the plurality of first serpentine beams. The second tube can include a plurality of parallel second slots that extend through the sidewall of the second tube perpendicular to a central axis of the second tube. The second slots can be arranged in rows of plural slots spaced radially from each other. Each row of second slots can be offset angularly from adjacent rows, wherein the second slots define the plurality of second serpentine beams.

According to another example, the first slots in each row can be equal in length and the second slots in each row can be equal in length.

According to another example, the first slots in each row can be of two different lengths arranged in an alternating fashion, and wherein the second slots in each row can be of two different lengths arranged in an alternating fashion.

According to another example, the steerable instrument can include a plurality of bending segments spaced axially along the length of the steerable instrument. The plurality of bending segments can include a first bending segment and a second bending segment. The first and second bending segments each can be formed by respective first serpentine beams formed in the first tube sidewall and second serpentine beams formed in the second tube sidewall that are axially aligned with each other.

According to another example, the first and second bending segments can be configured to deflect in different directions when actuated.

According to another example, the first and second bending segments can be separated by transition regions of the first and second tubes that are free from slots in the tube sidewalls.

According to another example, the first and second bending segments can be configured to deflect in opposite directions so that the steerable instrument assumes an S-shape when actuated.

According to another example, the angular positions of the first and second slots can be adjusted progressively about the central axis so that the bending segment bends out of plane in a helical manner when actuated.

According to another example, the first and second slots can include contact aids that limit bending motion of the bending segment in opposite bending directions.

According to another example, each contact aid can include a convexly shaped member protruding from a lateral beam member positioned in a correspondingly shaped concave receiver on an adjacent lateral beam member.

According to another example, each contact aid can have a dovetail configuration wherein the convexly shaped member comprises a dovetail pin and the concave receiver comprises a dovetail tail.

According to another example, at least a portion of the first and second slots can include stress relieving cutouts at an end thereof.

According to another example, the steerable instrument can include a first insertion shaft extending proximally from the first serpentine beam and a second insertion shaft extending proximally from the second serpentine beam. The first and second insertion shafts can be configured to be flexurally compliant to allow them to adopt to a tortuous path, omnidirectionally compliant so that they exhibit the same flexural stiffness regardless of the direction of loading, with axial stiffness and torsional rigidity to sufficiently transmit forces and torques from proximal ends of the insertion shafts to the distally located bending segment of the steerable instrument.

According to another example, the first insertion shaft can include a polymeric flexible tube positioned adjacent and connected to the first tube, a braided wire reinforcement layer overlying or embedded in the flexible tube, and polymer jacket that covers the braided wire and helps secure the braided wire on the flexible tube. The second insertion shaft can include a polymeric flexible tube positioned adjacent and connected to the second tube, a braided wire reinforcement layer overlying or embedded in the flexible tube, and polymer jacket that covers the braided wire and helps secure the braided wire on the flexible tube.

According to another example, the braid density of the braided wire can be varied along the length of the at least one of the first and second insertion shafts to adjust the flexibility, axial stiffness, and torsional rigidity of the insertion shaft along its length.

According to another example, the flexible tubes can be secured to their respective second tubes by one of embedding the tube in the flexible tube material, connecting the flexible tube to the outer tube with an adhesive, and forming the flexible tube as a thin jacket of material adhered to the outer tube.

According to another example, the first and second insertion shafts can include respective portions of the first and second tubes extending proximally of their respective serpentine beams and comprising a plurality of slots in their respective tube sidewalls. The slots can extend perpendicular to the central axis of the first and second tubes and can be arranged in rows that extend radially about the central axis. The slots can be configured so that the first and second insertion shafts are flexurally compliant to allow them to adopt to a tortuous path, omnidirectionally compliant so that they exhibits the same flexural stiffness regardless of the direction of loading, and axial stiff and torsionally rigid so that they transmit forces and torques from the proximal ends of the insertion shafts to the distally located bending segment of the steerable instrument.

According to another example, the pitch of the slots arranged along the first and second insertion shafts can be varied along the lengths of the first and second insertion shafts to adjust the flexibility, axial stiffness, and torsional rigidity of the first and second insertion shafts along their lengths.

According to another example, the steerable instrument can include a polymer jacket that covers the bending segment and prevents tissue from entering the slots.

According to another example, the slots defining the serpentine beams can be configured to be so narrow that tissue cannot enter the slots.

According to another example, the steerable instrument can be a compound steerable instrument including first and second steerable instruments. The second steerable instrument can be configured to extend through an inner lumen of the first steerable instrument.

According to another example, the surgical system can include a delivery device configured to be inserted and manipulated to position at a surgical site. The steerable instrument can be configured to be advanced through the delivery device and to have the bending segment positioned outside the delivery device with the remainder of the steerable instrument positioned in and supported by the delivery device.

According to another example, the delivery device can include an endoscope, a flexible endoscope, or another steerable instrument.

According to another example, the delivery device can be configured so that the steerable instrument, supported by the delivery device with the bending segment positioned outside the delivery device, can support a surgical instrument extending through the inner lumen of the nested tube structure for tissue manipulation at the surgical site.

### Brief Description of the Drawings

The foregoing and other features and advantages of the present disclosure will become apparent to one skilled in the art to which the present disclosure relates upon consideration of the following description of the invention with reference to the accompanying drawings, wherein like reference numerals, unless otherwise described refer to like parts throughout the drawings and in which:
Fig. 1 is a perspective view illustrating a planar serpentine beam element used for reference in describing the performance of a steerable instrument.
Fig. 2A is a perspective view illustrating a pair of tubes with serpentine beam elements, the tubes being configured to be nested together to form a steerable instrument that can be implemented in a surgical system.
Figs. 2B and 2C are perspective views illustrating the tubes of Fig. 2A concentrically nested to form a steerable instrument, and showing the manipulator in different deflected conditions.
Figs. 3A and 3B illustrate the parameters of the serpentine beam pattern implemented in the steerable instrument.
Figs. 4A and 4B are side and front/top views of a bending segment of the steerable instrument formed by the serpentine beam elements of the nested tubes, showing the bending segment in a non-actuated condition.
Figs. 4C and 4D are side and front/top views of the bending segment of Figs. 4A and 4B illustrating the bending segment in an actuated condition.
Fig. 5A is a planar view illustrating the cut pattern used to form a single serpentine beam element pattern in a tube used to form the steerable instrument.
Figs. 5B, C and D are top, side, and bottom views, respectively, of a tube including a single serpentine beam element pattern formed *via* the cut pattern of Fig. 5A.
Fig. 6A is a flat/planar view illustrating the cut pattern used to form a double serpentine beam element pattern in a tube used to form the steerable instrument.
Figs. 6B, C and D are top, side, and bottom views, respectively, of a tube with a double serpentine beam element resulting from the cut pattern of Fig. 6A.
Fig. 7A is a flat/planar view illustrating the cut pattern used to form a triple serpentine beam element pattern in a tube used to form the steerable instrument.
Figs. 7B, C and D are top, side, and bottom views, respectively, of a tube with a triple serpentine beam element resulting from the cut pattern of Fig. 7A.
Fig. 8A is a flat/planar view illustrating a cut pattern not part of the present invention used to form a quadruple serpentine beam element pattern in a tube used to form the steerable instrument.
Figs. 8B, C and D are top, side, and bottom views, respectively, of a tube with a quadruple serpentine beam element resulting from the cut pattern of Fig. 8A.
Fig. 9A is a flat/planar view illustrating the cut pattern used to form an n-serpentine beam element pattern with linear spacing in a tube used to form the steerable instrument.
Figs. 9B, C and D are top, side, and bottom views, respectively, of a tube with an n-serpentine beam element resulting from the cut pattern of Fig. 9A.
Fig. 10A is a flat/planar view illustrating the cut pattern used to form an n-serpentine beam element pattern with angular spacing in a tube used to form the steerable instrument.
Figs. 10B, C and D are top, side, and bottom views, respectively, of a tube with an n-serpentine beam element pattern with angular spacing resulting from the cut pattern of Fig. 10A.
Fig. 11A is a flat/planar view illustrating the cut pattern used to form a serpentine beam element pattern with increasingly reduced linear spacing in a tube used to form the steerable instrument.
Figs. 11B, C and D are top, side, and bottom views, respectively, of a tube with a serpentine beam element pattern with increasingly reduced linear spacing resulting from the cut pattern of Fig. 11A.
Fig. 12A is a flat/planar view illustrating the cut pattern used to form a serpentine beam element pattern with increasing cut fraction and slot overlap angle in a tube used to form the steerable instrument.
Figs. 12B, C and D are top, side, and bottom views, respectively, of a tube with a serpentine beam element pattern with increasing cut fraction and slot overlap angle resulting from the cut pattern of Fig. 12A.
Fig. 13A is a perspective view illustrating a pair of tubes configured to be nested together to form a steerable instrument, in which the tubes are configured with a single serpentine beam element pattern with a gradual angular offset.
Fig. 13B and 13C are perspective views illustrating the tubes of Fig. 13A concentrically nested to form a steerable instrument, and showing the actuator in different deflected conditions illustrating that the serpentine beam element pattern results in an out-of-plane bending profile.
Fig. 14A is a perspective view illustrating a pair of tubes configured to be nested together to form a steerable instrument, in which the tubes are configured with two discrete serpentine beam element patterns at spaced locations along their lengths.
Fig. 14B and 14C are perspective views illustrating the tubes of Fig. 14A concentrically nested to form a steerable instrument, and showing the actuator in different deflected conditions illustrating that the linearly spaced, discrete serpentine beam element patterns result in an S-bend when deflected.
Fig. 15 illustrates progressive stages of actuation of the steerable instrument of Figs. 14A and 14B showing how the S-bend evolves.
Figs. 16A-16D illustrate a tube with cut pattern used to form a serpentine beam element pattern configured to include contact aids for increasing torsional stiffness and preventing hyperextension in a tube used to form the steerable instrument, including a flat/planar view, as well as the resulting top, side, and bottom views of the tube, respectively.

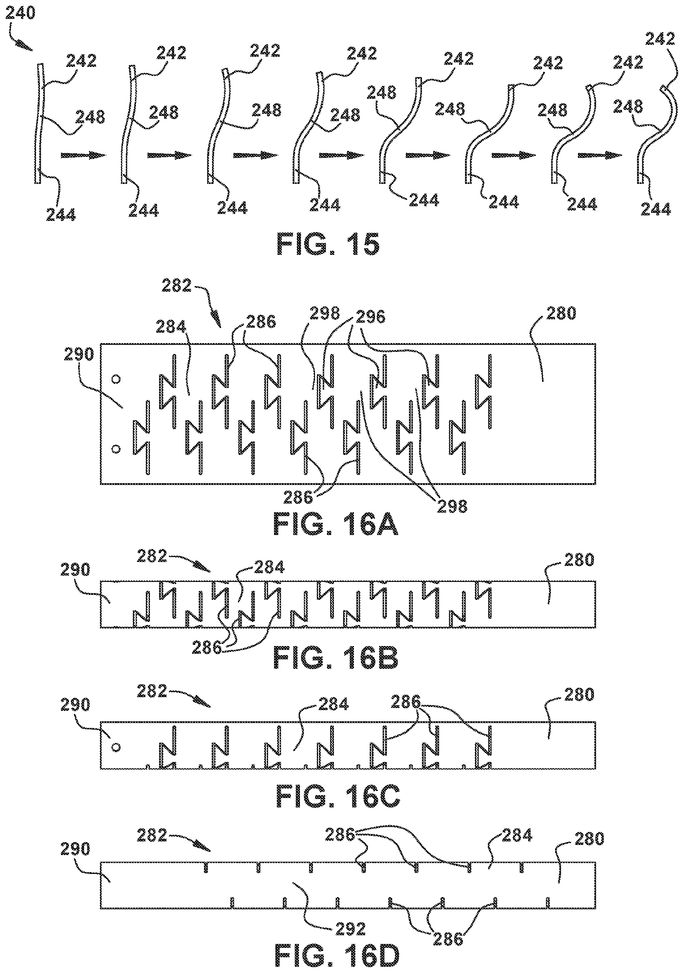

Fig. 16A is a flat/planar view illustrating the cut pattern used to form a serpentine beam element pattern configured to include contact aids for increasing torsional stiffness and preventing hyperextension in a tube used to form the steerable instrument.
Figs. 16B, C and D are top, side, and bottom views, respectively, of a tube including a single serpentine beam element pattern formed *via* the cut pattern of Fig. 16A.

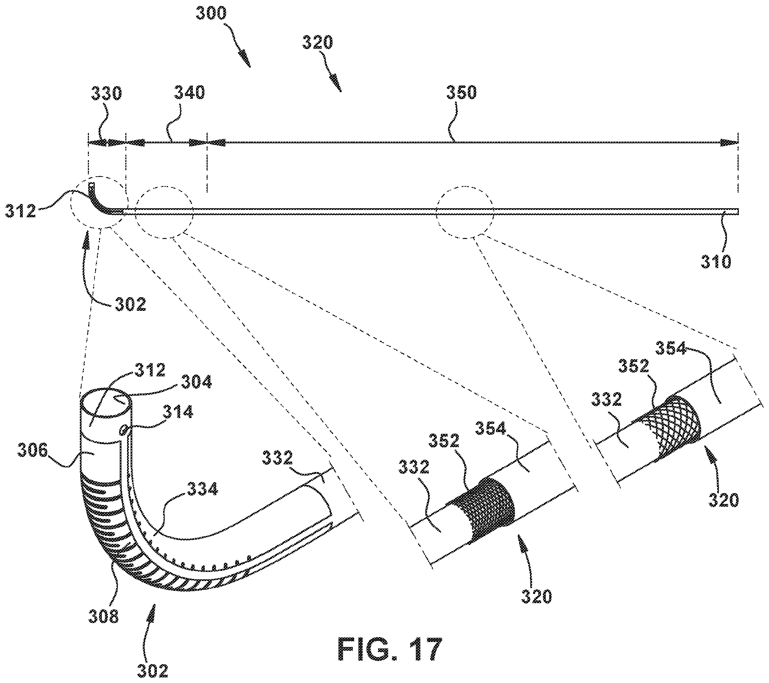

Fig. 17 illustrates the construction of different sections of a steerable instrument including an insertion shaft according to one example configuration.

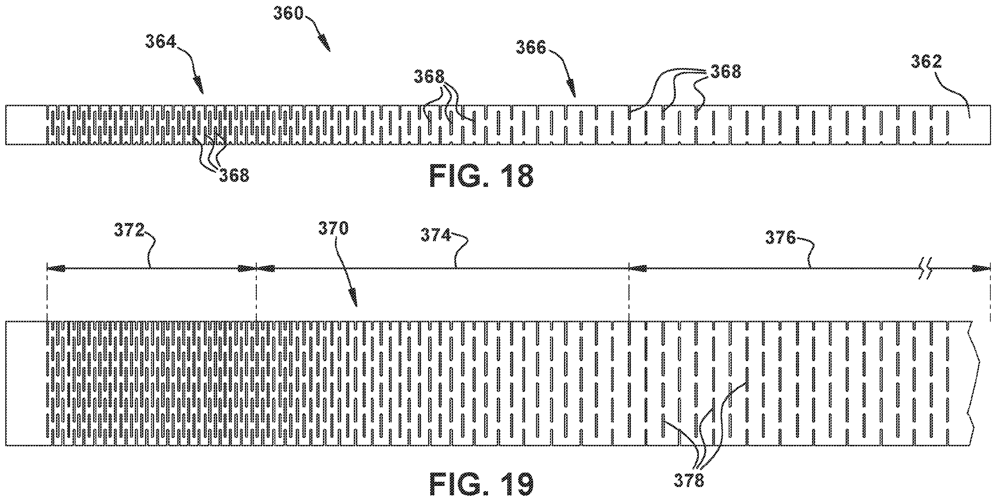

Fig. 18 illustrates an example tube configuration with a serpentine beam element and a laser-patterned insertion shaft.
Fig. 19 illustrates an example configuration for an insertion shaft with variable stiffness created through slot pitch variation.

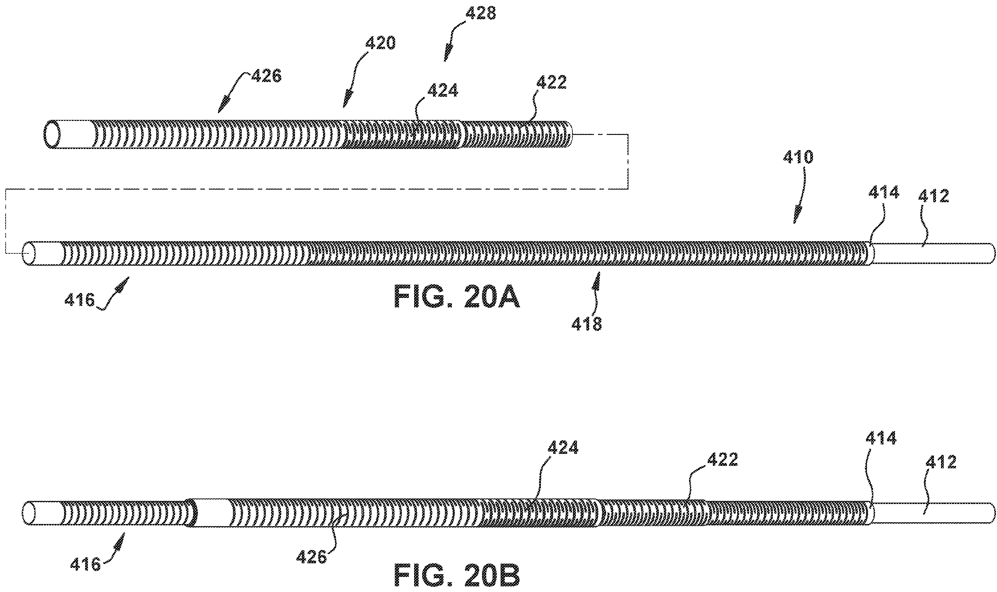

Figs. 20A and 20B illustrate an example configuration of a two steerable instruments that are nested concentrically to form a multiple degree-of-freedom steerable instrument system.

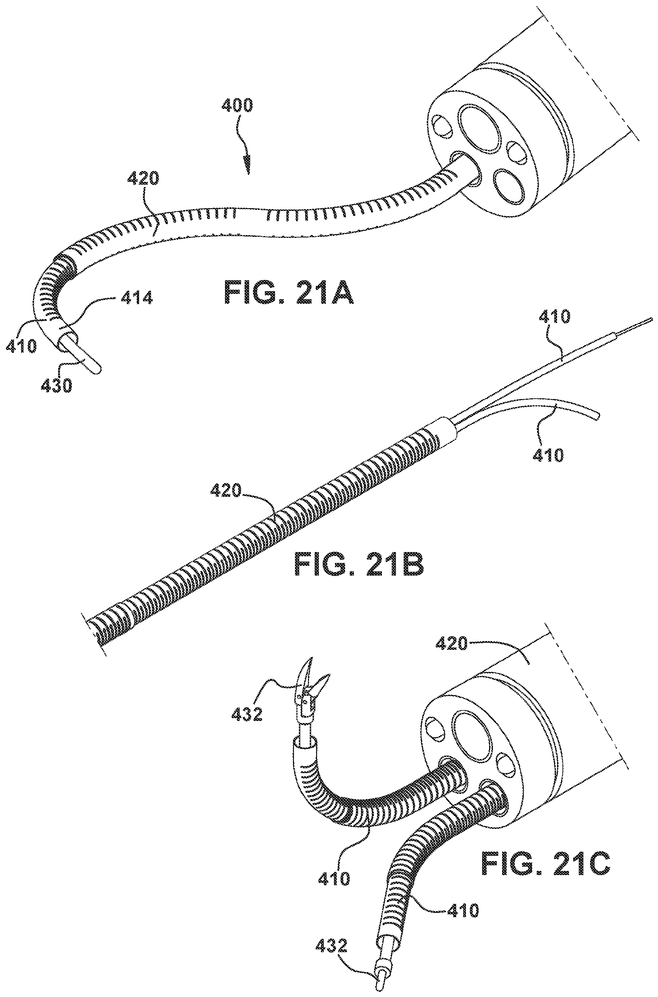

Fig. 21A illustrates an example configuration of a multiple degree of freedom steerable instrument system formed with two steerable instruments that are nested concentrically, with one of the nested steerable instruments carrying a surgical tool at its distal end.
Fig. 21B illustrates an example configuration of a multiple degree of freedom steerable instrument system formed with two steerable instruments that are carried side-by-side in a lumen of a third steerable instrument.
Fig. 21C illustrates an example configuration of a multiple degree of freedom steerable instrument system formed with two steerable instruments carried side-by-side in a flexible actuatable endoscope that acts as an outer steering structure, with the inner steerable instruments carrying surgical tools at their distal ends.

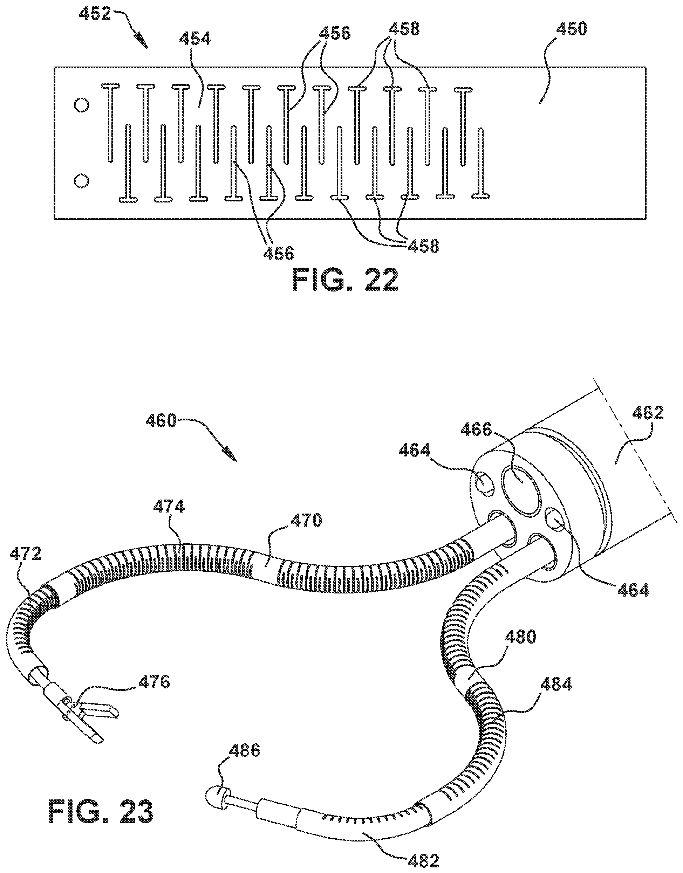

Fig. 22 is a flat/planar view illustrating an example configuration of a serpentine beam element in which the slots defining the serpentine beam element include stress reducing cut-outs at their ends.
Fig. 23 illustrates an example configuration of a bimanual endoscopic system including two multiple degree of freedom steerable instruments carried by a flexible endoscope.

### Detailed Description

### Steerable instrument with Serpentine Beam Elements

This disclosure relates to surgical systems that employ steerable instruments for use in endoscopic surgical procedures. The surgical systems can be manually-operated, where operation of the steerable instrument is performed through manual controls. The surgical systems can be robotic, where operation of the steerable instrument is performed *via* robots that are either controlled by a surgeon or pre-programmed to perform specific routines. The surgical systems can also be a combination of manually-operated and robotic.

In this disclosure, the steerable instruments are agonist-antagonist steerable instruments constructed from a pair of tubes that are nested concentrically, one inside the other. The tubes include slots that are cut through their respective sidewalls to define one or more longitudinally extending serpentine beam elements that offset the neutral axis of each tube away from the geometric axis. When the steerable instrument is assembled, the beam elements are aligned axially and positioned facing in radial directions that differ angularly from each other, such as a radially opposing fashion in which the beam elements are face 180 degrees from each other, to define a bending segment. The tubes are interconnected distally of the bending segment, and the bending segment can be actuated in an agonist-antagonist manner by exerting an axial differential push/pull force on the tubes. Actuation causes the bending segment to bend in opposite directions based on the direction in which the axial push/pull force is applied. This configuration exploits the flexural properties of serpentine beam elements in a cylindrical form factor to create the bending segment.

In use, the bending segment can be used to steer the tubes and/or manipulate tissue through the use of surgical tools carried by the tubes. In this description, the assembled concentric tube structure is shown and described as a steerable instrument, where the bending segment is used to control or "steer" the instrument. The bending segment is typically, but not necessarily always, positioned at the distal tip of the instrument.

The "steering" function of the bending segment can serve many purposes: to deliver and use surgical tools, to access difficult to reach locations in the patient anatomy, and to guide or direct other tools/instruments to perform a surgical procedure. For implementations where the instrument is used to deliver and use surgical tools, the bending segment can be used to manipulate tissue *via* the tools. In other implementations, the tubes, being hollow, can be used for fluid/drug delivery and/or suction. For implementations where the instrument is used to provide access to surgical locations, the hollow construction of the tubes can allow for the introduction of wires (e.g., for ablation lasers), control cables (e.g., for mechanical tool actuation), and tools themselves (e.g., surgical probes, baskets). The actuatable steerable instrument can also be used to carry and guide other steerable instruments. The term "steerable instrument," as used herein, is meant to encompass steerable concentric tube structures with bending segments formed from serpentine beam elements configured to implement any of these functions in any combination.

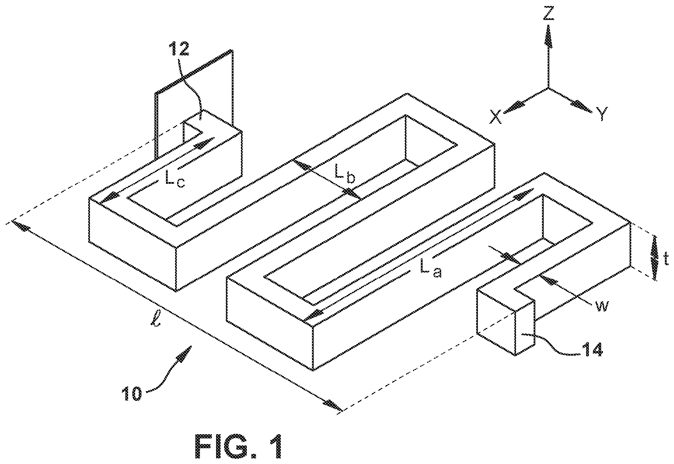

Serpentine beams are commonly employed in microelectromechanical systems (MEMS) to create flexural elements that can undergo precision zero-backlash deformation in multiple prescribed axes. As shown in Fig. 1, a serpentine beam element 10 includes a single beam of material, mechanically grounded at a fixed end 12, that meanders back-and-forth to a guided or working end 14. The back-and-forth configuration of the beam element 10 increases the effective length for bending, lending to flexibility in both in-plane and out-of-plane directions.

The serpentine beam element 10 can be configured to exhibit much higher axial and bending compliance than a single (straight) beam with the same cross-sectional area, defined by cross-sectional dimensions, i.e., width *w* and thickness *t,* and effective length *l*. The compliance can be further 'tuned' by careful design of the serpentine profile, i.e., by modifying the parameters beam width (*Lₐ*), beam pitch (*L_{b}*), and a beam base dimension (*L_{c}*) in Fig. 1. In the MEMS space, serpentine beams are commonly used as bearings in inertial measurement units and nano-positioners, as well as in other applications where the small size scale prohibits the use of more conventional bearing elements, such as ball bearings, bushings, gears, *etc.*

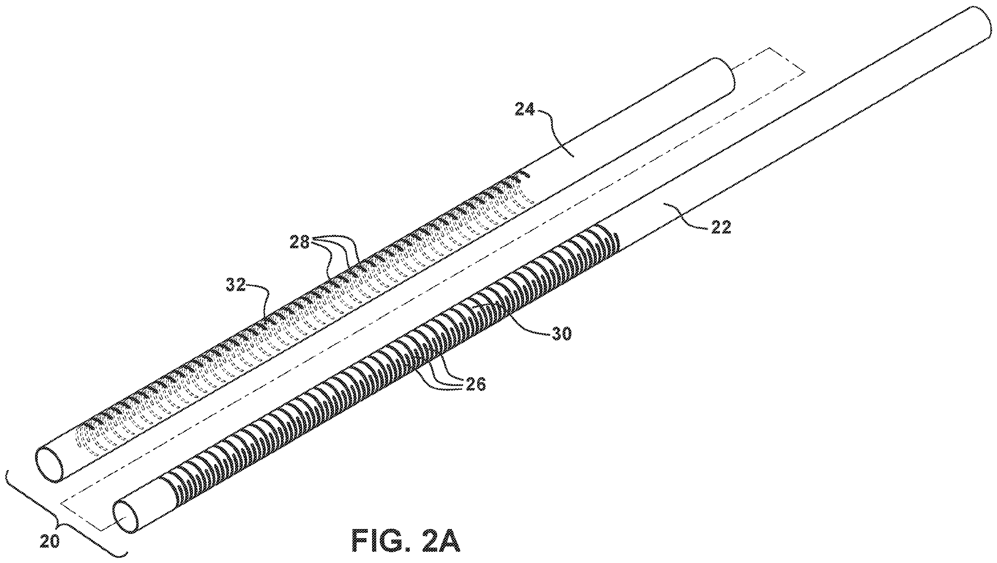

As described herein, steerable medical instruments leverage the flexural properties of monolithic serpentine beam elements to enforce preferential bending in millimeter-scale tubes, where two or more tubes with these characteristics are combined to create preferential bending in multiple directions. The surgical system can implement the steerable instruments *via* manual control, robotic control, or a combination of manual and robotic control. Several example configurations of these steerable instruments that can be implemented in a surgical system are disclosed in the following paragraphs, a first of which is shown in Figs. 2A-2C.

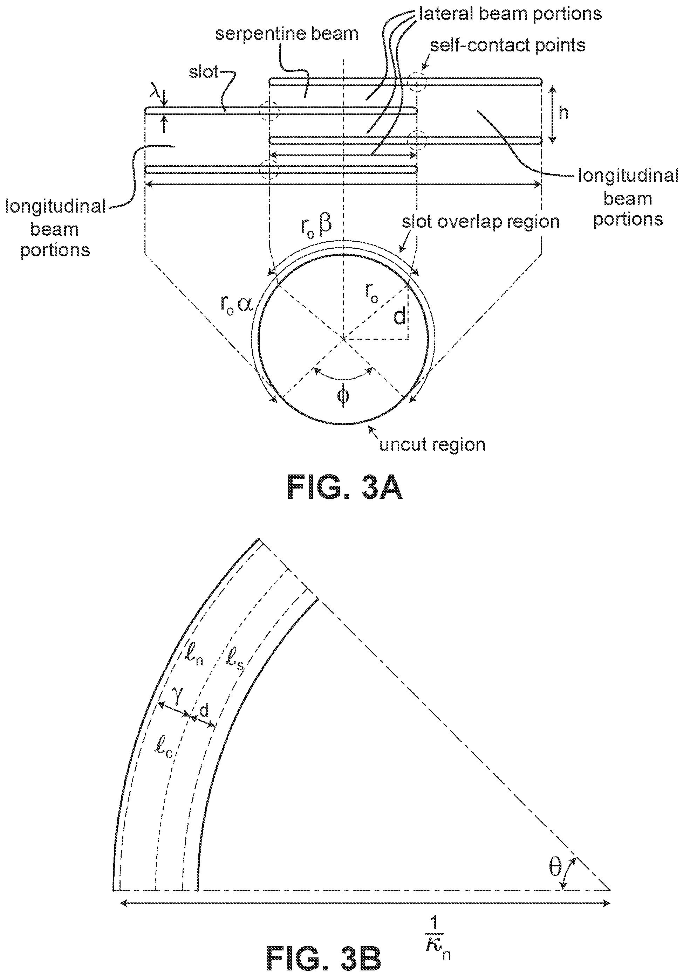

In Fig. 2A, a steerable instrument 20, includes an inner tube 22 and an outer tube 24 that are configured to be nested, with the inner tube being inserted into the outer tube, as indicated generally by the dashed line in Fig. 2A. Each tube 22, 24 includes one or more slots 26, 28, respectively, that form serpentine beam elements 30, 32, respectively. Unlike traditionally planar serpentine beam elements commonly used in MEMS, the serpentine beam elements 30, 32 formed in the tubes 22, 24 are curved, as they are wrapped around a cylinder. The beam elements 30, 32 are formed through the creation of the slots 26, 28 that are micromachined in the sidewalls of the tubes 22, 24. In Figs. 2A-2C, each tube 22, 24 includes a single serpentine beam element 30, 32 formed from a series of slots 26, 28 that extend through radial portions of the tube sidewall in a direction perpendicular to the central axis of the tube. The serpentine beam elements 30, 32 therefore include lateral beam portions that extend radially along the tube sidewall between adjacent parallel slots 26, 28 and longitudinal beam portions that extend axially along the tube sidewall and interconnect ends of adjacent lateral beam portions in an alternating fashion (see, Fig. 3A).

The slots 26, 26 are spaced from each other lengthwise along the tube and are offset angularly, i.e., rotated relative to each other in a back-and-forth manner so as to form the serpentine pattern of their respective beams 30, 32. The slots 26, 28 and, thus, the beam elements 30, 32, can vary in configuration, e.g., size, spacing, number, dimensions, *etc.* Formed in the sidewall of the tubes 22, 24, the cylindrical serpentine beam elements 30, 32 locally reduce the bending stiffness on side of the tube in which they are formed. As a result, this offsets the neutral bending axis of the tube away from the its geometric axis, which promotes preferential bending of the tubes 22, 24 in the area of the beams 30, 32.

In an assembled condition of the steerable instrument 20, the inner tube 22 is inserted inside the outer tube 24 and advanced so that the beam elements 30, 32 are aligned with each other axially and rotated so that the serpentine beam elements 30, 32 face in directions that differ angularly from each other, such as the radially opposite directions shown in Figs. 2A-2C. Once the inner tube 22 is installed in the outer tube 24 with their beam elements 30, 32 aligned axially and facing radially opposite each other, the tubes are interconnected or fastened to each other distally of the of the beam elements. This connection or fastening can, for example, be at the distal end 34 of the instrument 20.

In the assembled condition of the steerable instrument 20, the beam elements 30, 32 define a deflectable portion or bending segment 40 that is actuatable to cause bend to form along its length. Actuation of the bending segment 40 is effectuated through relative axial motion between the nested tubes 22, 24 in a push/pull manner. Application of a push force on the tubes is where the inner tube 22 is pushed into the outer tube 24, toward its connection with the outer tube. In the example configuration of Figs. 2A-2C, the push force on the inner tube 22 urges it toward the distal end 34 of the steerable instrument 20. Application of a pull force on the tubes is where the inner tube 22 is pulled out of the outer tube 24, away its connection with the outer tube. In the example configuration of Figs. 2A-2C, the pull force on the inner tube 22 urges it away from the distal end 34.

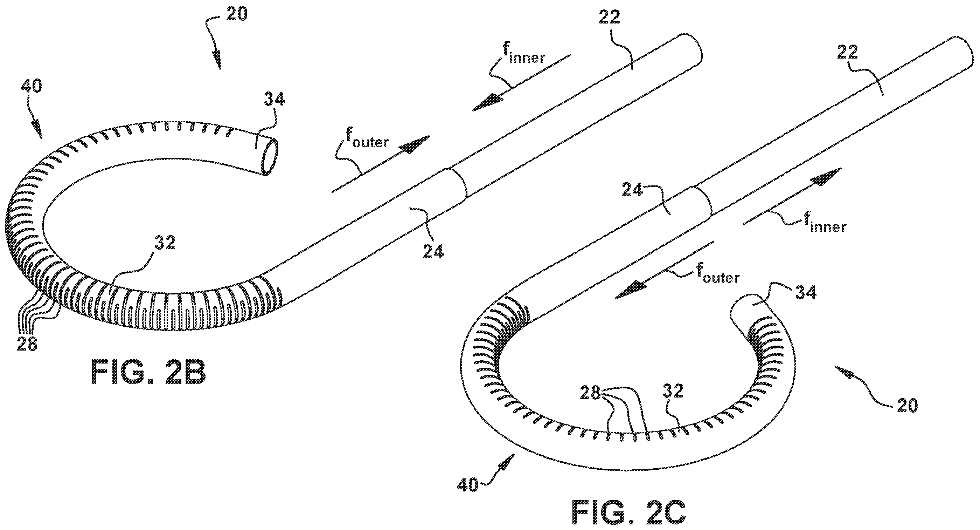

Of course, application of the axial push/pull forces is not limited to those applied to the inner tube 22 alone. The push/pull forces can be applied to the inner tube 22, the outer tube 24, or both the inner and outer tubes. For example, a push force can be established by applying differential axial forces on the tubes 22, 24 (fᵢₙₙₑᵣ, fₒᵤₜₑᵣ) in net opposite directions toward each other, as shown in Fig. 2B. As another example, a pull force can be established by applying differential axial forces on the tubes 22, 24 (fᵢₙₙₑᵣ, fₒᵤₜₑᵣ) in net opposite directions away each other, as shown in Fig. 2C.

As shown in Figs. 2B and 2C, the bending segment 40 is configured to be bi-directionally actuatable, that is, push actuation causes the bending segment to deflect and bend in a first direction, and pull actuation causes the bending segment to deflect and bend in an opposite direction. This is because the tubes 22, 24 are nested together with the serpentine beam elements 30, 32 aligned axially, and anti-aligned radially (facing radially opposite) such that the neutral axes of each tube are in diametric opposition. With the tubes 22, 24 fastened at the distal end 34 of the steerable instrument 20, the bending segment 40 bends in response to the differential axial force (fᵢₙₙₑᵣ, fₒᵤₜₑᵣ) applied at the proximal to the bending segment. The bending occurs about the combined neutral axis of the tubes 22, 24. The direction of bending is reversible simply by reversing the direction of the differential axial force applied to the proximal end, as shown in Figs. 2B and 2C.

In the example configuration of Figs. 2A-2C, and in other example configurations described herein, the serpentine beam elements of the inner and outer tubes are axially aligned so as to completely overlap each other and rotated so as to face radially opposite each other. The configuration of the bending segment formed by the serpentine beam elements can depart from this arrangement. For example, the axial alignment of the serpentine beam elements can be configured so as to partially overlap and/or so that the beam elements face in different radial directions not necessarily opposite, i.e., 180 degrees from each other. This can be advantageous in implementations where it may be desirable for the bending section deflect in a non-planar direction. In such a non-180 degree implementation, the bending section, when actuated, can produce a combination of bending and twisting that creates a nonplanar helical bend.

### Nitinol as a Tube Material

The tubes used to construct the steerable instruments described herein can be constructed of Nitinol, a superelastic metal alloy of Nickel and Titanium. While serpentine beam profiles may be leveraged in tubes constructed from any number of common engineering materials, the steerable instruments disclosed herein, being configured to manipulate tissue and operate surgical tools, require a certain combination of properties that render many conventional materials less desirable than Nitinol. While materials other than Nitinol, such as stainless steel, have proved to be suitable for forming a steerable instrument, Nitinol has proven to be the best. This is not to say that other materials, such as stainless steel or even some plastics, are not suitable, just that Nitinol is ideal in terms of performance.

Conventional metals, such as steel, titanium, or aluminum, feature high Young's moduli (200 GPa, 100 GPa, and 70 GPa, respectively), but have very low yield strains (0.1-0.2%), limiting the amount of deflection that tubes created with these materials can undergo before permanent plastic deformation and failure. These metals are therefore ideal for surgical instrument/tool implementations that require strength, rigidity, and stiffness, such as cutters, grippers, probes, *etc.,* but are less suitable for those that require flexibility and resilience.

Conversely, many medical-grade plastics commonly used in the construction of flexible medical tubes and other instruments (such as polyether block amide (PEBA) materials, Nylon materials, or Polyimide materials) have much higher yield strains (10-100%), but have low Young's moduli (0.1-2.0 GPa), severely limiting the achievable stiffness of tubes created with these materials. These plastics are therefore ideal for implementations that require strength, flexibility, and resilience, but are less suitable for those that require rigidity and stiffness.

Nitinol combines the most favorable properties of these conventional metal and plastic materials. Nitinol offers excellent performance in terms of high flexural stiffness (Young's modulus 40-80 GPa) and the ability to undergo large strain before plastic deformation (yield strain >8%). These properties are essential for creating steerable instruments capable of generating large distal forces for deflecting adjunct medical tools and manipulating patient tissue, while also generating large displacements for high dexterity and a large reachable workspace. This combination of physical properties make Nitinol an ideal material from which to construct the steerable instruments described herein.

In applications where the steerable instruments are configured to deliver medical tools, fluid flow, or suction/irrigation, while themselves being delivered through a flexible delivery platform (such as a flexible endoscope, or another steerable instrument), maintaining a very thin wall is of utmost importance in order to maximize the available space inside the steerable instrument through which to deliver tools, fluids, or suction. Maintaining thin walls introduces challenges related to torsional and axial stiffness, as well as kink resistance. The ideal material has a high Young's modulus, high shear modulus, and high yield strain. Nitinol incorporates all of these properties.

The superelastic nature of Nitinol (displaying a region of low-stiffness behavior between 1% and 8% strain) reduces the necessary actuation force for a given distal deflection when compared to similar metals, which greatly facilitates the use of the material in hand-actuated devices that require mechanical input from a human to generate the proximal force necessary to deflect the tip of the device.

### Beam Profile Generalizability

The configuration of the serpentine cut pattern creates a 'spine' of uncut material on one side of the tube, and a serpentine pattern on the opposing side. Various parameters of the serpentine cut pattern can be modified to alter the bending properties of the tube. These parameters, shown in Figs. 3A and 3B, include the following:
- Pitch - the longitudinal spacing between subsequent serpentine elements (h). When all other parameters are held constant, a higher pitch (shorter spacing) increases bending compliance and reduces torsional stiffness.
- Slot Overlap Angle - the 'width' of the serpentine meander normalized to the tube circumference, expressed as an angle (rₒβ). The overlapping angle between adjacent slots. When all other parameters are held constant, a larger overlap angle increases bending compliance.
- Cut Fraction - total angle swept by the slot (rₒα). When all other parameters are held constant, a larger cut fraction increases bending compliance.
- Number of Parallel Serpentines - number of serpentine elements. A larger number of parallel serpentines reduces bending compliance and increases torsional stiffness.
- Slot Width - width of each individual slot (λ). When all other parameters are held constant, a larger slot width creates a higher bending angle for the steerable instrument.

From a purely kinematic standpoint, one may calculate the maximum bending angle of a single serpentine tube member when given the design variables listed above. The total width of the pattern is given by the angular parameter *a,* while the tube circumference is uncut in the region of *φ* = 2*π - α,* leaving a solid backbone. The dimensions of the backbone relative to the size of the tube impact its stiffness and must be selected such that the tube is not too stiff for actuation but still robust. The backbone dimensions also determine the location of the neutral axis with respect to the centerline; this distance γ can be found with the following relationship: $γ = \frac{{γ}_{o} {A}_{o} - {γ}_{i} {A}_{i}}{{A}_{o} - {A}_{i}} ,$ (1)
where, γₒ,γᵢ, *Aₒ,* and *Aᵢ* are the neutral axis locations and areas for the circular sectors formed by the outer and inner radii of the uncut region, respectively: $\begin{matrix}{γ}_{o} = \frac{4 {r}_{o} sin \left(\frac{ϕ}{2}\right)}{3 ϕ} \\ {γ}_{i} = \frac{4 {r}_{i} sin \left(\frac{ϕ}{2}\right)}{3 ϕ} \\ {A}_{o} = \frac{{r}_{o}^{2} ϕ}{2} \\ {A}_{i} = \frac{{r}_{i}^{2} ϕ}{2} ,\end{matrix}$ (2)
where *rₒ* and *rᵢ* are the outer and inner radii of the tube, respectively.

The slot overlapping angle β determines how far from the centerline of the tube that the slots come into self-contact, which is the physical limit for tube bending. This distance from the centerline is given by $d = {r}_{o} cos \left(\frac{β}{2}\right) ,$ (3)
as seen in Fig. 3B. Next, we must find the relationship between slot closure and the change in length of the device. Here, we assume that the change in length of the entire length of the sheath is proportional to the change in length for one set of slots. In other words, for each slot spacing h, the maximum change in length for that segment of tube is the height of the slot λ. Therefore, we have $Δ l = \left(\frac{λ}{h}{l}_{n}\right) ,$ (4)
where ℓ*ₙ* is the length of the sheath neutral axis (this length does not change and is a design input). As seen in the right half of Fig. 3B, there are three length parameters all related by the same deflection angle, θ. *ℓ_{c}* is the centerline length of the device, and *ℓₛ* is the length of the device at the serpentine pattern closure points. We have that ${l}_{s} = {l}_{n} - Δ l = {l}_{n} - \frac{λ}{h} {l}_{n} = {l}_{n} \left(1-\frac{λ}{h}\right) ,$ (5)
allowing us to relate these lengths using the common bending angle: $θ = {κ}_{n} {l}_{n} = {κ}_{c} {l}_{c} = {κ}_{s} {l}_{s} = {κ}_{s} {l}_{n} \left(1-\frac{λ}{h}\right) ,$ (6)
where *κₙ, κ_{c},* and *κₛ* are the neutral axis, centerline, and serpentine closure point curvatures, respectively. We use the geometry of the tube to define the relationships between the curvatures: ${κ}_{s} = \frac{1}{{r}_{n} - γ - d} = \frac{{κ}_{n}}{1 - {κ}_{n} γ - {κ}_{n} d} ,$ (7)
where *rₙ* = 1/*κₙ* and γ is the distance from the centerline to the neutral axis, as solved using Equations (1) and (2). Using this curvature relationship and Equation (5), we can solve for the maximum bending angle of the tube as dictated by closure and self-contact of the serpentine slots: $θ = \frac{λ {l}_{n}}{h \left(γ+{r}_{o} cos\left(\frac{β}{2}\right)\right)} ,$ (8)

Using this derived relationship, we can calculate the maximum bending angle of the patterned tube. Alternatively, one can solve for any other of the desired parameters if given a desired bending angle or curvature. The bending segment of the steerable instrument can therefor be tailored to the specific procedure for which it intended.

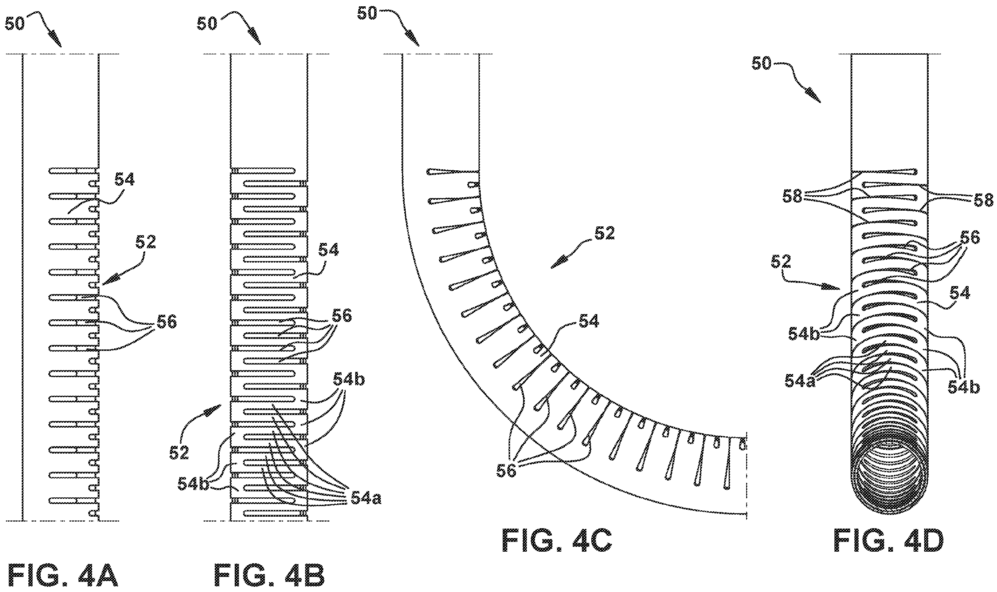

Figs. 4A-4D illustrate how the serpentine beam parameters described above define the bending profile of a steerable instrument 50 with a bending segment 52 formed by a serpentine beam element 54 is defined by a series of slots 56 cut through the tube sidewall. Figs. 4A and 4B show front and side views of the steerable instrument 50 in an unactuated condition where the bending segment 52 is not deflected and takes on a straight configuration that is coaxial with adjacent portions of the tube. In this condition, the serpentine beam element 54 is non-deflected and the configuration/spacing of the slots 56 is uniform across the bending segment 52.

As best shown in Fig. 4B, The serpentine beam elements 54 includes lateral beam portions 54a that extend radially along the tube sidewall between adjacent slots 56 and longitudinal beam portions 54b that extend axially along the tube sidewall and interconnect ends of adjacent lateral beam portions in an alternating fashion.

Figs. 4C and 4D shows front and side views of the steerable instrument 50 in an actuated condition where the bending segment 52 is deflected and takes on a curved configuration. In the actuated condition of the bending segment 52, the serpentine beam elements 54 deflect at the longitudinal beam portions 54b and adjacent lateral beam elements 54a engage each other at touch points 58 at ends opposite the deflection point. From this, it can be seen how parameters of the tube, such as the pitch λ and the slot width h define the max curvature of the bending segment 52.

Engagement of the touch points 58 define the fully deflected condition of the bending segment 52. When the bending segment 52 is in the fully deflected condition, the differential axial forces applied to the tubes maintain the bending segment in the fully deflected condition. Increasing the differential axial forces applied to the tubes above that required to reach full deflection will not cause further deflection because the engaging touch points 58 prevent this from happening. Advantageously, however, increasing the differential axial force will increase the rigidity or stiffness of the bending segment 52. This can be especially beneficial where the steerable instrument 50 is used for tissue manipulation. In this instance, the rigid/stiff bending segment 52, supported by a delivery device, can manipulate tissue without being deflected itself as a result of reaction forces with the tissue.

The delivery device through which the steerable instrument 50, or any of the various steerable instruments disclosed herein are delivered, can vary. For example, the steerable instrument 50 can be delivered through a rigid endoscope or a flexible endoscope. As another alternative, the steerable instrument 50 can be delivered through another steerable instrument constructed in accordance with one or more of the example configurations disclosed herein. As a further alternative, a serpentine beam bending segment 52 can be implemented as a built-in component of a delivery device, such as a flexible endoscope. In this case, the bending segment would be made larger to facilitate the delivery of surgical tools and other components through the inner lumen of the bending segment. This could, for example, enable the delivery of a camera or other device .

### Different Serpentine Configurations

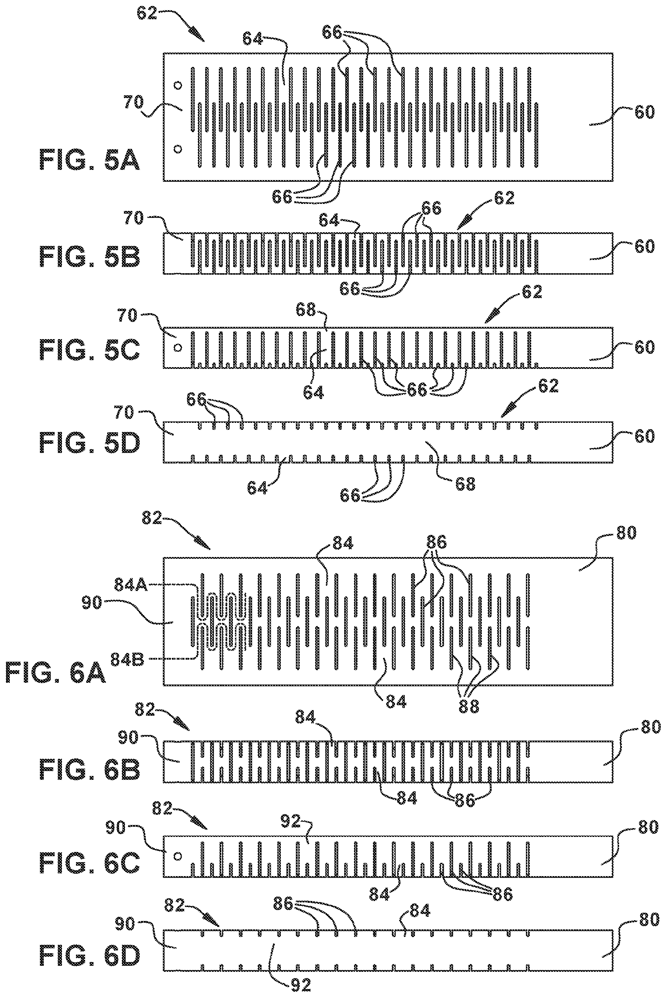

Figs. 5A-12D illustrate a variety of example serpentine beam element configurations that can be implemented in the tubes of a steerable instrument to form a bending segment. Figs. 5A-12D illustrate these various serpentine beam patterns in a single tube, with the understanding that the pattern will be implemented in two tubes, which will be assembled as described above to form a steerable instrument and the bending segment thereof. The variations in the configuration of the serpentine beam element(s) determines the bending properties of the bending segments they form. In each of these example configurations, the bending segment illustrated as being located at a distal tip of the tube. This bending segment location is not, however, limiting. Any of the bending segment configurations disclosed in Figs. 5A-12D can be applied at any location along the length of the steerable instrument.

### Single-Serpentine

A tube 60 with a bending segment 62 formed from a single-serpentine beam pattern is shown in Figs. 5A-5D. Fig. 5A shows a flat/planar plan view of a distal end of the tube, with the tip 70 being indicated by circles where the tube is configured for connection to another nested tube (not shown). The bending segment 62 could, however, be located at any position along the tube 60.

In Fig. 5A, the tube 60 is shown as if the tube 60 was cut longitudinally along the spine 68 (see Fig. 5D) and flattened, to aid in visualization of serpentine beam 64 and the slots 66 that define the beam. Top, side, and bottom plan views of the tube 60 are shown in Figs. 5B-5D, respectively. This single serpentine beam element 64 runs longitudinally on the low stiffness side of the tube 60, opposite the spine 68. The single serpentine beam pattern of Figs. 5A-5D offers the highest flexural compliance capability (a single serpentine beam with the longest meander) and lowest torsional stiffness. This pattern is most appropriate in small (<1.5mm tube diameter) medical devices with low torsional payload requirements, such as applications requiring steering of energetic tools (laser fibers) or tools which only require axial force (Nitinol baskets).

Some common design rules to follow when designing the single-serpentine tube 60 are as follows.
- *Slot Overlap Angle* (*rₒβ*): The overlap angle for a single serpentine design should reside between 10-180 degrees.
- *Cut Fraction* (*rₒα*): The cut fraction should reside between 190-320 degrees. Any less and the tube will be too stiff (limiting overall deflection), any more will render the uncut 'spine' too fragile.
- *Slot Width (A):* 25 µm-200 µm. On the lower end, this is limited by the kerf (or spot size) of the laser used to manufacture the serpentine beam elements, which is typically 25 µm. The low end value could vary as manufacturing methods improve. The high end value could also increase, for example, if tubes larger than those set forth above are employed, or in other situations where the application demands a larger gap/increased per-gap bending.

### Double-Serpentine

A tube 80 with a bending segment 82 formed from a double-serpentine beam pattern is shown in Figs. 6A-6D. Fig. 6A shows a flat/planar plan view of a distal end of the tube, with the tip 90 being indicated by circles where the tube is configured for connection to another nested tube (not shown). The bending segment 82 could, however, be located at any position along the tube 80.

Fig. 6A shows a flat/planar plan view, as if the tube 80 was cut longitudinally along the spine 92 (see Fig. 6D) and flattened, to aid in visualization of serpentine beams 84 and the slots 86 that define the beams. Top, side, and bottom plan views of the tube 80 are shown in Figs. 6B-6D, respectively. The double-serpentine pattern shown in Figs. 6A-6D, is created by two slots 86 of equal length, followed by a single slot 88 rotationally offset from the previous two slots, effectively creating two serpentine beam elements 84 (mirrored about the tube centerline) running in parallel. The individual serpentine beams of the double serpentine beam pattern is identified at 84A and 84B in Fig. 6A. The double serpentine beam configuration has good flexural compliance and moderate torsional stiffness, and is best suited for moderate-bore applications (1.5-3mm tube diameter) where interaction forces with tissue require higher torsional stiffness.

### Triple-Serpentine

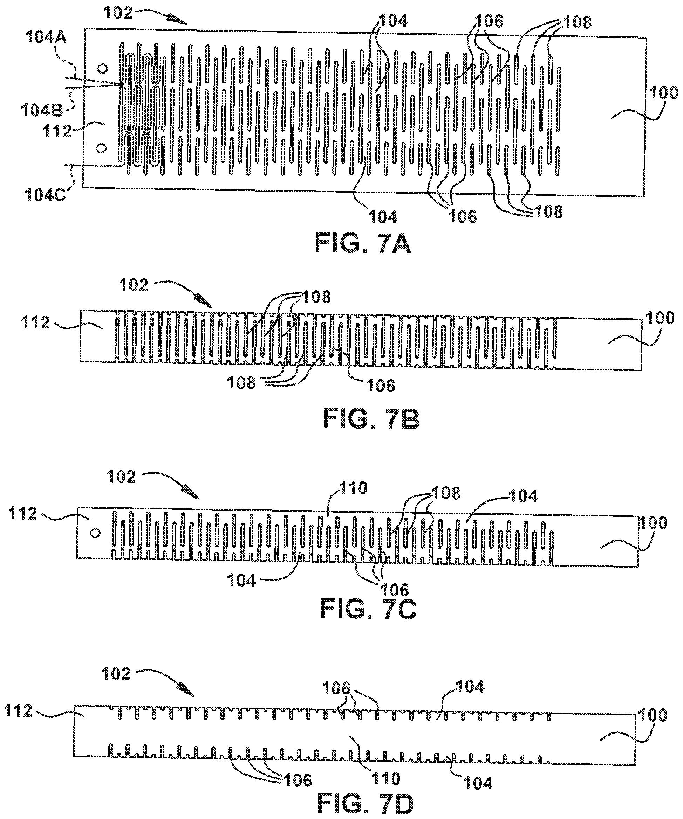

A tube 100 with a bending segment 102 formed from a triple-serpentine beam pattern is shown in Figs. 7A-7D. Fig. 7A shows a flat/planar plan view of a distal end of the tube, with the tip 112 being indicated by circles where the tube is configured for connection to another nested tube (not shown). The bending segment 102 could, however, be located at any position along the tube 100.

Fig. 7A shows a flat/planar plan view, as if the tube 100 was cut longitudinally along the spine 110 (see Fig. 7D) and flattened, to aid in visualization of serpentine beams 104 and the slots 106 that define the beams. Top, side, and bottom plan views of the tube 100 are shown in Figs. 7B-7D, respectively. The triple-serpentine pattern shown in Figs. 7A-7D includes three serpentine beams 104 running in parallel on the low-stiffness side of the tube. This is created by two cuts 106, 108 of unequal length, staggered longitudinally, effectively creating three serpentine beams 104 running in parallel. The individual serpentine beams 104 of the triple-serpentine beam pattern is identified at 104A, 104B, and 104C in Fig. 7A.

The triple-serpentine beam profile of the tube 100 offers moderate flexural compliance and high torsional stiffness, and is best suited for larger-bore applications (2-3mm tube diameter) where tissue manipulation has the potential to create large off-axis loads, generating high torsional forces. The three serpentine beams 104 are arranged around the tube in 100 degree increments. The beam 104B opposite the solid spine 110 typically has a larger overlap angle than the two "side" beams 104A and 104C.

### Quadruple-Serpentine

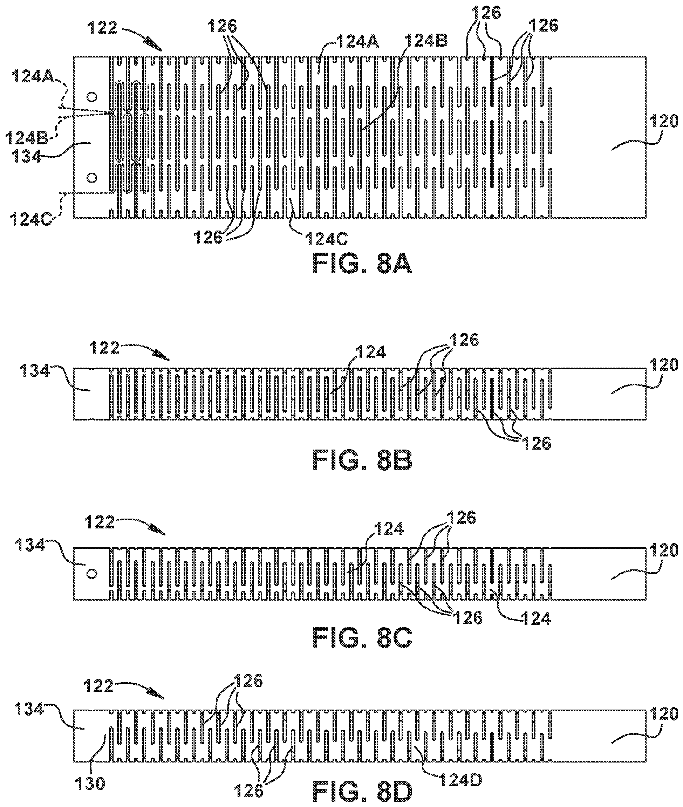

A tube 120 with a bending segment 122 formed from a quadruple-serpentine beam pattern is shown in Figs. 8A-8D. Fig. 8A shows a flat/planar plan view of a distal end of the tube, with the tip 134 being indicated by circles where the tube is configured for connection to another nested tube (not shown). The bending segment 62 could, however, be located at any position along the tube 120.

Fig. 8A shows a flat/planar plan view, as if the tube 120 was cut longitudinally along the spine 130 (see Fig. 8D) and flattened, to aid in visualization of serpentine beams 124 and the slots 126 that define the beams. Top, side, and bottom plan views of the tube 120 are shown in Figs. 8B-8D, respectively. The quadruple-serpentine beam pattern shown in Figs. 8A-8D includes three serpentine beams 124 running in parallel on the low-stiffness side of the tube, opposite the spine 120. This is created by two cuts 126 of equal length, staggered longitudinally, effectively creating three serpentine beams 124 running in parallel. Three of the individual serpentine beams 124 of the quadruple-serpentine beam pattern are identified at 124A, 124B, and 124C in Fig. 8A. The fourth serpentine beam pattern 124D extends along the spine 130, as shown in Fig. 8D.

In an example not part of the present invention, the quad-serpentine beam pattern shown in Figs. 8A-8D differs from the previous patterns of Figs. 5-7 in that the solid spine is replaced with a spine 130 formed as another serpentine beam 132 with much higher flexural stiffness than the other three serpentine beams 124A, 124B, and 124C. The higher flexural stiffness along the spine 130 is owed to the smaller overlap angle of the slots 126 along the spine.

The quadruple-serpentine configuration of the tube 120 exhibits moderate flexural compliance with high torsional stiffness, making it most suited for larger (2-3.5mm diameter) steerable instruments required to carry stiffer tools and manipulate tissue that produces large torsional loads. As with the triple-serpentine pattern, the four beams are spaced around the tube in 90 degree increments so that there is one serpentine beam on each of the four "sides" of the tube. The two beams with the highest flexural stiffness (beam 132) and lowest flexural stiffness (beam 114B), which are created with the lowest and higher overlap angle, respectively, are arranged opposite each other on the spine and top of the tube 120, while the two side beams (beams 114A and 114C) have the same overlap angle for uniform flexural stiffness in that direction.

As an additional benefit of the quadruple-serpentine configuration, the flexural stiffness of the of the tube 120 can be independently controlled in two perpendicular planes - the actuation plane and the plane perpendicular to the actuation plane/tube cross section plane - while still shifting the neutral axis towards one side of the cross section. For instance, these bi-directional flexural stiffnesses can be made equal for a single tube. This is useful in reducing parasitic torsional deformation, which can result from unequal stiffnesses and misalignment of tubes. It can also simplify modeling and control for the multi-tube steerable instruments described herein.

### n-Serpentine

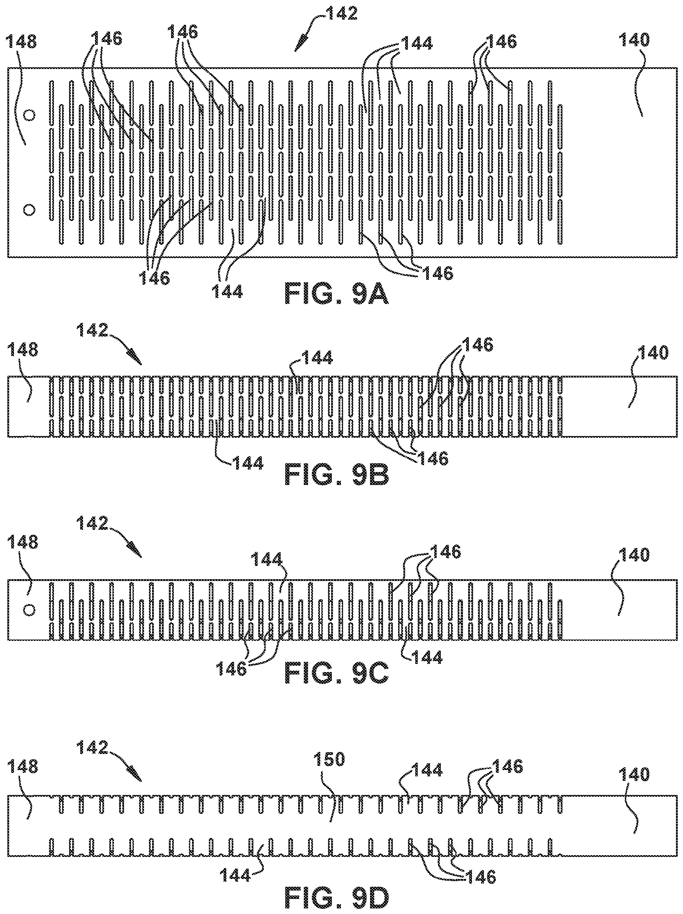

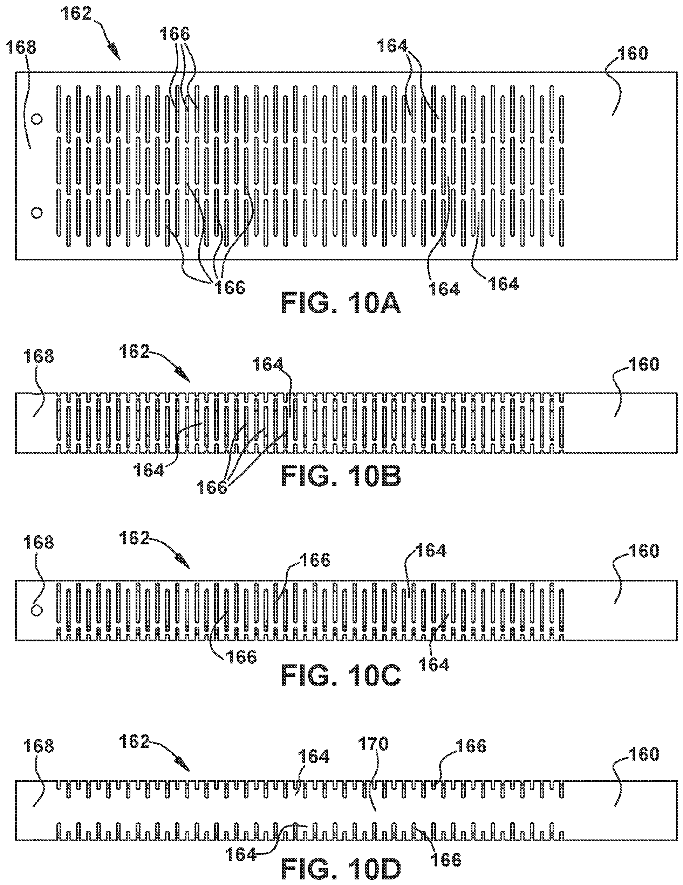

The number of parallel serpentine elements can theoretically increase indefinitely. Similarly, the serpentine beam patterns can be placed at arbitrary angular locations about the tube. The number of parallel serpentine elements is ultimately limited by the tube circumference and manufacturing limitations. Examples of tubes 140, 160 with n-serpentine beam element bending segments 142, 162 are shown in Figs. 9A-9D and 10A-10D, respectively.

Figs. 9A-10D are flat/planar plan view, as if the tubes 140, 160 were cut longitudinally along their respective spines 150, 170 and flattened, to aid in visualization of the slots that define the serpentine beams. Figs. 9 and 10 show flat/planar plan views of distal ends of the tubes, with their tips 148, 168 being indicated by circles where the tube is configured for connection to another nested tube (not shown). The bending segments 142, 162 could, however, be located at any position along the tubes 140, 160.

In Figs. 9A-9D, the slots 146 are arranged in groups of three cuts that are linearly aligned about the circumference of the tube 140 and are of equal length and spacing about the tube circumference. The groups are staggered, i.e., rotated relative to each other, about the tube axis so that adjacent groups are rotationally offset from each other. This can be referred to as angular spacing of the slots 146 or groups of slots. The angularly spaced arrangement of the slots 146 in groups creates serpentine beams 144 that run in parallel to each other along the length of the tube 140.

Similarly, in Figs. 10A-10D, the slots 166 are arranged in groups of three cuts that are linearly aligned about the circumference of the tube 160 and are of equal length and spacing about the tube circumference. The groups are staggered, i.e., rotated relative to each other, about the tube axis so that adjacent groups are rotationally offset from each other. This can be referred to as angular spacing of the slots 166 or groups of slots. The angularly spaced arrangement of the slots 166 in groups creates serpentine beams 164 that run in parallel to each other along the length of the tube 160.

The configurations of the serpentine beams 144, 164 in Figs. 9A-10D is due to the difference in the angular spacing of the slots 142, 162. The slots 142, 162 and the pitch or spacing between the slots is the same. It is the angular spacing between the groups of slots 142, 162 that differs between the tubes 140, 160 of Figs. 9A-9D and 10A-10D. In Figs. 9A-9D, the space between each pair of slots 142 in a group is centered on the adjacent slots. In Figs. 10A-10D, the space between each pair of slots 162 in a group is positioned near an end of the adjacent slots. As a result, the beams 144 in Figs. 9A-9D differ in configuration from the beams 164 of Figs. 10A-10D.

### Methods for Variable Curvature

For the tubes of Figs. 5A-10D, the configurations of the serpentine beam elements are defined by the slot cut patterns. The length and spacing of the slot(s) in each group, i.e., at each axial location along the length of the bending segment/tube, as well as variations in the relative angular positions of the slots/spaces at each axial location, contributes to the resulting serpentine beam(s), which bend uniformly along the length of the bending segment. All of the cut patterns disclosed in Figs. 5-10 can be modified to increase or decrease the curvature of the bending segment, for example, by increasing or decreasing the length of the bending segment, by increasing or decreasing the number of bends in the serpentine beam patterns, by increasing or decreasing the pitch of the serpentine beam patterns, or a combination of these modifications.

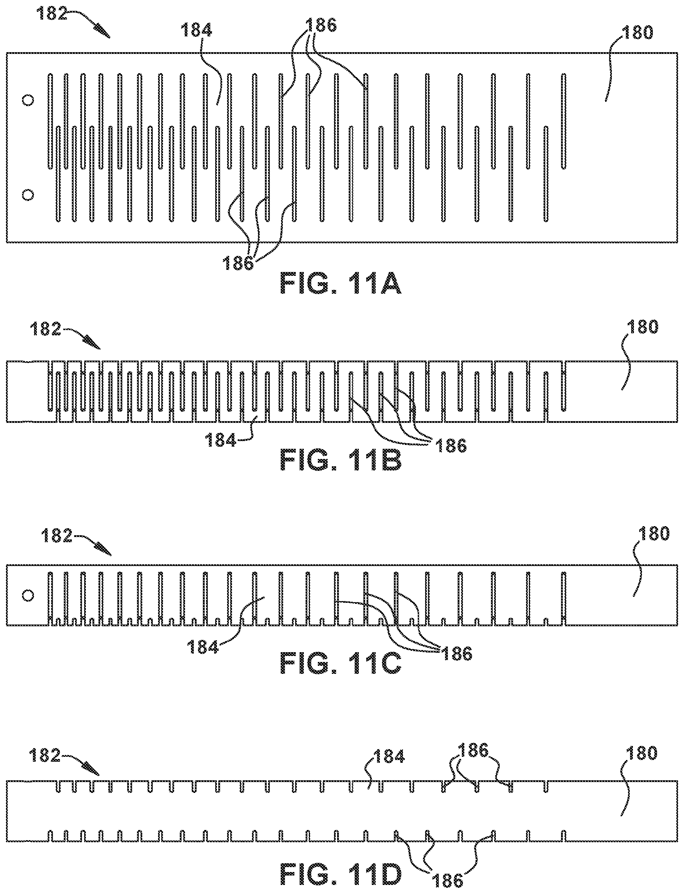

Example configurations of the bending segment that create a variable bending segment curvature are illustrated in Figs. 11A-12D. Variable bending segment curvatures can be implemented, for example, to produce a preferential tip-first bending or to correct for friction accumulation between the two nested tubes. A variable curvature can be induced through a variation in pitch, cut fraction, slot overlap angle, or variations in a combination of these parameters.

For example, in Fig. 11, a variable curvature in a tube 180 with a single-serpentine beam element bending segment 182 is implemented *via a* linear reduction in pitch. In this example, all of the slots 186 are uniform in length, width, and rotational spacing, with the pitch between the slots being reduced progressively, proximal to distal along the bending segment. As a result, the width of the serpentine beam element 184 is reduced progressively, proximal to distal. With the distal end of the bending segment 182 configured to coincide with the distal tip of the tube 180, this creates 'tip first' bending because the bending stiffness of the serpentine beam element 186 at the distal end of the bending segment is lowest and increases incrementally in the proximal direction.

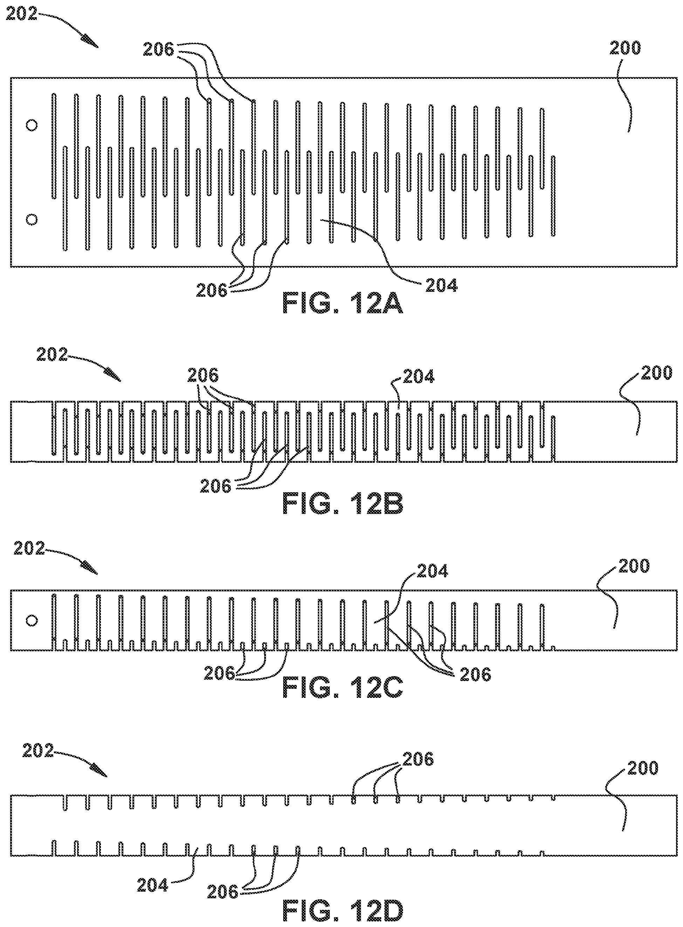

As another example, in Fig. 12, a variable curvature bending segment 202 in a single-serpentine beam element tube 200 is implemented with a uniform pitch where the lengths of the slots 206 and the overlap of adjacent slots increases from proximal to distal along the bending segment. As a result, the serpentine beam element 204 has a linear reduction in pitch spacing from the proximal to distal end. This creates 'tip first' bending because the bending stiffness of the serpentine beam element at the distal end of the bending segment is lowest and increases incrementally in the proximal direction.

### Methods for Inducing Out-Of-Plane Bending

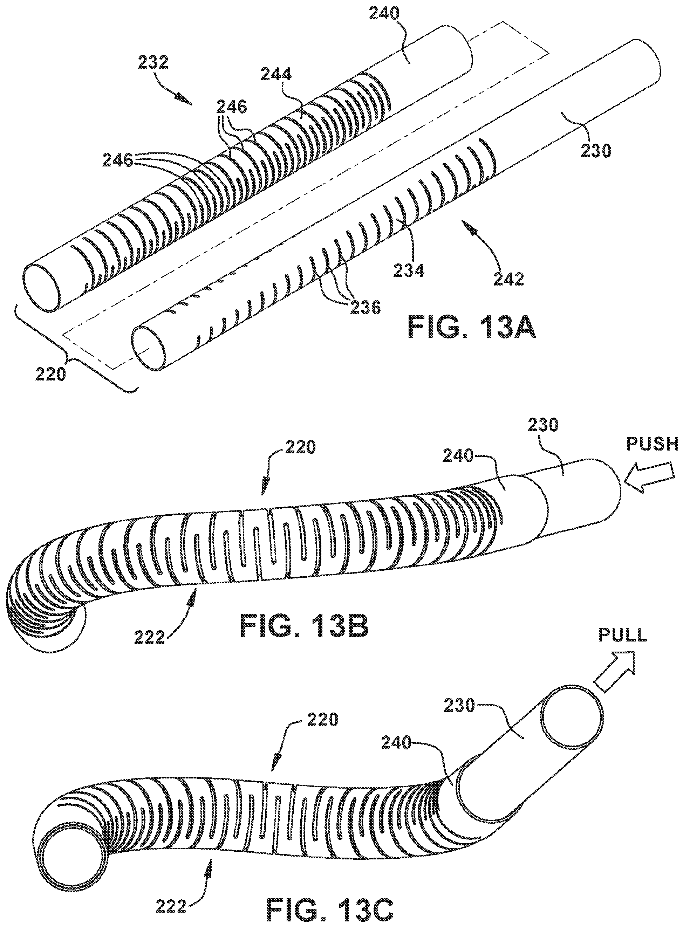

In certain applications, it may be desirable to have the steerable instrument 'twist' out-of-plane to prevent the instrument form colliding with itself at large deflection angles. This is shown in the example steerable instrument configurations of Figs. 13A-13C. Referring to Figs. 13A-13C, a steerable instrument 220 includes a bending segment 222. In Figs. 13A-13C, the bending segment 222 is located at a distal end 224 of the steerable instrument 220. The bending segment 222 could, however, be located at any desired position along the length of the tube 220.

The steerable instrument 220 includes an inner tube 230 and an outer tube 240. The inner tube 230 has a bending segment 232 that includes a serpentine beam element 234 defined by a series of slots 236 cut through the tube sidewall. The outer tube 240 has a bending segment 242 that includes serpentine beam element 244 defined by a series of slots 246 cut through the tube sidewall. In each tube 230, 240, the slots 236 and 246 are offset angularly and progressively along the length of their respective bending segments 232, 242. In an assembled condition of the steerable instrument 220, which is shown in Figs. 13B and 13C, the inner tube 230 is positioned inside the outer tube 240 with their respective bending segments 232, 242 aligned axially and rotated 180-degrees to face in radially opposite directions.

The steerable instrument 220 is configured for actuation *via* the application of an axial push-pull force through on the tubes 230, 240 to cause the bending segment 222 to deflect in opposite directions, as shown in Figs 13B and 13C. In Fig. 13B, the bending segment 222 is actuated *via* a push force in which the inner tube 230 is pushed into the outer tube 240, as indicated by the arrow labeled PUSH in Fig. 13B. In Fig. 13C, the bending segment 222 is actuated *via* a pull force in which the inner tube 230 is pulled out of the outer tube 240, as indicated by the arrow labeled PULL in Fig. 13C.

Through the push-pull actuation, the steerable instrument 220 can be actuated in opposite directions be controlling the push-pull force applied to the tubes. As shown in Figs. 13B and 13C, the angular offset between the slots 236, 246 and beams 234, 244 causes neutral axis of each tube 230, 240 to twist about the geometric axis of the tube in a helical fashion. As a result, when the bending segment 222 is actuated it bends out-of-plane in a helical manner, as shown in the figures. When the tubes 230, 240 are configured with bending segments 232, 242 having their slots 236, 246 and beams 234, 244 configured identically, the bending segment 222 will actuate identically, out-of-plane in a helical fashion, but in opposite directions depending on the push-pull force applied to the steerable instrument 220, as shown in Figs. 13B and 13C.

The magnitude of the out-of-plane bending is determined by the amount of angular offset between subsequent slots 236, 246. This capability can be beneficial for high deflection (>180 degrees) to avoid self-collision of the steerable instrument 220 distal end with the proximal end, or in applications where the instrument is configured to deliver an endoscopic camera to avoid obfuscating the camera's field-of-view with the proximal section of the bending segment 222 itself.

### Methods for Locally Modifying the Bend Direction

In some applications, it may be advantageous for a steerable section of a steerable instrument to incorporate a bend direction reversal. This can be important in applications where two steerable instruments are delivered through a flexible endoscope in a bimanual configuration. In this configuration, the steerable instrument could, for example, be configured to bend slightly out of camera view, and then back into view, to achieve triangulation.

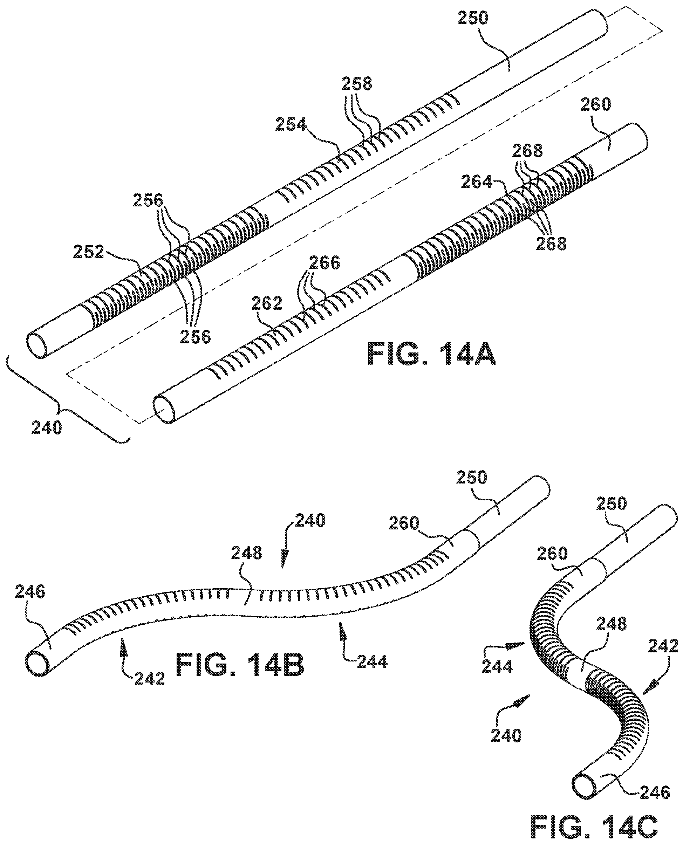

A steerable instrument 240 with multiple bending segments 242, 244 is shown in Figs. 14A-14C. One bending segment 242 is located distally on the steerable instrument 240 and the other bending segment 244 is located proximally. The steerable instrument 240 includes an inner tube 250 installed in an outer tube 260, with the tubes being interconnected at the distal end or tip 246 of the steerable instrument. The inner tube 250 includes two serpentine beam elements 252, 254 that are defined by two discrete sections of slots 256, 258, respectively, cut through the sidewall of the tube and arranged in a manner consistent with any of those disclosed herein, such as the manner disclosed in Figs. 2A-2C. The outer tube 260 includes two serpentine beam elements 262, 264 that are defined by two discrete sections of slots 266, 268, respectively, cut through the sidewall of the tube and arranged in a manner consistent with any of those disclosed herein, again, such as the manner disclosed in Figs. 2A-2C. When the steerable instrument 240 is assembled, the beam elements 252 and 262 align with each other to form the bending segment 242, and the beam elements 254 and 264 align with each other to form the bending segment 244.

The bending segments 242, 244 are rotated angularly relative to each other and can be separated by a solid section of tubing that defines a transition region 248, as shown in Figs. 14A-14C. Alternatively, the bending segments could meet in a transition region where the series of slots 254, 264 defining the bending segment 242 gradually reduces in the proximal direction to the end point (for example, in the manner disclosed in Figs. 11A-11D), and the series of slots 256, 266 defining the bending segment 244 gradually increases in the proximal direction from the end point, in a reverse manner. In the example configuration of Figs. 14A-14C, the bending segments 242, 244 are rotated 180 degrees with respect to each-other so that the steerable instrument 240 takes on an S-shaped configuration when the bending segments are actuated.

Fig. 15 illustrates the incremental actuation of the bending segments 242, 244 of the steerable instrument 240. As shown in Fig. 15, the bending segments 242, 244 are configured to produce bends in opposite directions, separated by the solid transition region 248. As the bending segments 242, 244 are actuated, their curvatures increase until, in the final frame, they become fully deflected. From this, it can be seen that the reach of the steerable instrument 240 can be configured for the intended application.

### Increasing Stiffness through Contact Aids

Steerable instruments can be configured to exhibit a sudden and drastic change in flexural or torsional stiffness beyond a desired bending or twisting angle through contact aids and self-collision. This may be desirable to prevent over- extension of the steerable instrument, or to create a stable, high-stiffness platform for other tools and devices passed through the steerable instrument. This can be advantageous, for example, where the tip of the steerable instrument is guided to a surgical site where a tool passed through the inner lumen of the steerable instrument is used to perform a surgical procedure that requires as rigid support as possible, such as for excising tissue. In these scenarios, a delivery device, such as an endoscope (rigid or flexible) can deliver the steerable instrument to the general area of the surgical site and the steerable instrument is navigated to the precise location of the surgical site. While the endoscope can certainly provide the requisite degree of rigid support, this can be moot if the steerable instrument cannot. Accordingly, the steerable instrument can incorporate features in the form of contact aids that produce an improvement in the rigidity with which the curvature of the steerable instrument is held when actuated.

An example of this is shown in Figs. 16A-16D. Fig. 16A shows a flat/planar plan view of a distal end of a tube 280 including a bending segment 282 located at a distal end 290 of the tube, which is indicated by circles where the tube is configured for connection to another nested tube (not shown). The bending segment 282 could, however, be located at any position along the tube 280. The flat/planar plan view is as if the tube 280 was cut longitudinally along the spine 292 (see Fig. 16D) and flattened, to aid in visualization of a serpentine beam 284 and the slots 286 that define the beam. Top, side, and bottom plan views of the tube 280 are shown in Figs. 16B-16D, respectively.

The slots 286 define a serpentine beam 284 that includes a plurality of beam elements 294 that extend in a back and forth manner, as described herein. As best shown in Fig. 16A, the slots 286 are configured with contact aids in the form of dovetail features that prevent the slots from opening indefinitely, thereby increasing the stiffness of the steerable instrument at a prescribed bending angle. In the configuration of Figs. 16A-16D, the slots 286 define a pin 296 on one side of each beam element 294 and a tail 298 positioned opposite the pin. As a result, the pin 296 of each beam element 294 is positioned in a tail 298 of the directly adjacent beam element 294.

The pins 296 and tails 298 have a generally trapezoidal configuration, with the pins being separated from the tail in which it is received by the width of the slot 286 by which they are formed. The trapezoidal configurations of the pins 296 and tails 298 create an interference in both bending directions, providing hard stops not just for closing, but also opening the bending segment 282. Thus, for example, if a surgical tool delivered to the surgical site through a steerable instrument in which the concentric tubes include the dovetail features described above, operation of the tool that results in a force that acts to open the curvature of the bending segment 282 will be blocked by the interaction of the pins 296 and tails 298.

As an additional feature, the pins 296 and tails 298 also serve to increase the torsional stiffness by limiting the amount of relative twist between the serpentine beam elements 294. Thus, if a twisting force is exerted on the bending segment 282, such as due to use of a surgical tool delivered to the surgical site through the steerable instrument, rotation of the beam elements 294 relative to each other will be blocked by the interaction of the pins 296 and tails 298.

It will be appreciated that the contact aids are not limited to the dovetail features shown in Figs. 16A-16D. For example, similar or identical results could be achieved *via* slots configured to define a keyhole shaped feature protruding from a beam element and positioned in a correspondingly keyhole shaped receiver on an adjacent beam element. In fact, similar or identical results could be achieved *via* a convexly shaped member protruding from a beam element that is positioned in a correspondingly shaped concave receiver on an adjacent beam element. Furthermore, the slots could be configured to produce more than one contact aid, such as two or more dovetail features, on each beam element, which can further improve the rigidity of the bending segment and resistance to rotation in response to working forces encountered during use.

### Methods for Delivery through Flexible Endoscopes

When the steerable instruments disclosed herein are configured to be delivered through another flexible delivery mechanism, such as another steerable sheath, a flexible endoscope, or an endoscope with a built-in serpentine beam bending segment, the distal working end of the steerable instrument where the bending segment(s) incorporating the serpentine beam element(s) are located will be separated from the proximal actuation end of the steerable instrument by a considerably long distance, such as 70cm-2000cm. Additionally, the portion of the steerable instrument between the proximal actuation end and the distal working end will often follow a curvilinear path. To enable the transmission of differential forces and common-mode torques from the proximal actuation end, through the curvilinear profile, to the steerable distal working end, the steerable instrument can be delivered *via* an insertion shaft that provides preferential flexural, axial, and torsional properties. The insertion shaft can take the place of the proximal portion of the outer tube of the steerable instrument, with the distal end of the outer tube including the bending segment is maintained as a Nitinol tube that is connected to the insertion shaft.

When being delivered through another flexible delivery system such as a flexible endoscope, it is important for the insertion shaft to display a suitable degree of flexural compliance to allow it to adopt the tortuous shape enforced by the curvilinear profile of the delivery system. In addition, the flexural stiffness of the insertion shaft must be omnidirectionally compliant (i.e., it must exhibit the same flexural stiffness regardless of the direction of loading) in order to prevent the steerable instrument from settling into preferred (lowest stiffness) configurations, which can create torsional deadbands and produce 'snapping' effects, which are described below. Finally, the insertion shaft must exhibit suitable axial stiffness and torsional rigidity to sufficiently transmit forces and torques from the proximal actuation system to the distal working end of the steerable instrument.

Torsional 'snapping' occurs due to a sudden release of energy as the tube rapidly transitions from one low-energy (low stiffness) state to another low-energy state as the tube is rotated while constrained within a curvilinear path. When this happens, the steerable tip is observed to quickly rotate between two angles in a sudden and uncontrollable fashion as the tube snaps from one low-energy state to another. If the insertion shaft is configured to be omnidirectionally compliant (i.e., the flexural stiffness is the same regardless of the direction of loading), snapping can be avoided.

Snapping can also occur in tubes with high torsional compliance due to friction at the interface between the curvilinear path constraint and the tube. As the tube is rotated at the proximal end, friction is generated between the tube and the path interface, which is a function of the tube's flexural stiffness (the stiffer the tube, the higher the friction). If the tube also has low torsional stiffness, torsional energy will accumulate within the tube (torsional windup) until a certain critical rotation angle, at which point the stored torsional energy overcomes interfacial friction and is suddenly released, causing a rapid and uncontrolled rotational unwinding at the distal end. This can be avoided by creating tubes with low flexural stiffness and high torsional stiffness.

### Fixation to flexible polymer tube insertion shafts

Referring to Fig. 17, a steerable instrument 300 includes a bending segment 302 formed by concentric inner and outer tubes 304, 306, respectively, with serpentine beam elements 308 formed therein. The bending segment 302 and the beam elements 308 that help define the bending segment, can have any of the configurations described herein. Where the steerable instrument 300 differs from other configurations described herein is that each of the inner and outer tubes 304, 306 instead of extending the length of the steerable instrument 302 from proximal end 310 to distal end 312, occupy only a distal section 330 of the instrument. Instead, each of the inner and outer tubes 304, 306 of the bending segment 302 are connected to an insertion shaft 302 that extends from the tubes/distal section 330 to the proximal end 310.

The insertion shafts 320 are themselves tubular in construction and configured to be nested, one inside the other, and offer the function in terms of delivering and controlling the operation of the bending segment 302 in manners identical to those described herein with regard to the other steerable instruments configurations. The difference, of course, lies in their configurations and material constructions. While the insertion shaft 320 shown in Fig. 17 is that connected to the outer tube 306 of the bending segment 302, it should be understood that the identity of the tube - outer or inner - is immaterial, as the construction of the insertion shaft is identical in both instances. The description of the insertion shaft 320 is therefore descriptive of the insertion shaft of the outer tube 306 and the insertion shaft of the inner tube 304.

The insertion shaft 320 can have a variety of constructions using different materials and/or components selected to provide desired performance characteristics, such as shaft-to-shaft friction, tube stiffness (axial and torsional), and flexibility. In the example configuration of Fig. 17, the insertion shaft 320 includes a flexible polymer tube 332 that is connected to the tube 306, a braided wire 352 wrapped outside the flexible tube for reinforcement, and a polymer jacket 354 that covers the braided wire. The construction of the insertion shaft 320 can be configured to vary in configuration along its, such that it exhibits variable stiffness properties. This can be achieved continuously through a gradual increase or reduction in braided wire density and/or the incorporation of longitudinal braid members. This can also be achieved discretely through the usage of different jacket materials with varying stiffness at different locations along the tube. In general, with all other properties held constant, an insertion shaft with a higher braid density will exhibit higher flexural compliance, torsional stiffness, and kink resistance, and an insertion shaft with lower braid density will exhibit higher axial stiffness.

As shown in Fig. 17, the steerable instrument 300 includes three sections, each of which is configured to provide the structural and functional properties set forth in the previous section so that the steerable instrument 302 can function as described herein. Specifically, the instrument 300 includes the distal section 330, which includes the bending segment 302, a transition section 340, and a proximal section 350. The components of the insertion shaft 320 (i.e., the flexible tube 332, the braided wire 352, and the jacket 354) can have portions that occupy all three sections 330, 340, 350 of the steerable instrument 300.

As mentioned previously, the bending segment 304 is formed by one or more serpentine beam members in the tubes 304, 306, in accordance with any of the example configurations described herein. The concentric tubes 304, 306 forming the bending segment 302 are interconnected at the distal end 312 of the instrument. The distal tip 306 can also include a radio-opaque marker 314 for visualization during imaging.

At the distal section 330, the steerable instrument 300 is affixed to the flexible polymeric tube 332 via an adhesive bending segment or by being embedded in the polymer forming the tube. The flexible tube 332 can be a single medical-grade polymeric material (e.g., Nylon 12, PEBA, or Polyimide) or a composite material consisting of multiple disparate materials, with or without the braided wire 352 reinforcement layer. The flexible tube 332 can be configured to exhibit the required material properties - high omnidirectional flexural compliance, high axial stiffness, high torsional stiffness - through material selection the incorporation and configuration of the braid-reinforcement layer. As shown in the detail of the distal section 330, the bending segment 302 of the steerable instrument 300 can be embedded in polymeric material, either of the tube 332 itself or in a different polymeric material. In either case, the material in which the bending segment 304 is embedded can be formed as a thin jacket 334, so as not to impact bending segment dexterity or actuation. Thus, at the distal section 330 of the steerable instrument 300, the insertion shaft 320 can include only this thin jacket 334 that covers the bending segment 302.

At the proximal section 350 of the steerable instrument 300, the insertion shaft 320 includes the flexible tube 332 and the braided wire 352 for reinforcing the tube 332. In the proximal section 350, the braided wire 352 can be coarsely braided (i.e., a low braid density), which produces high axial stiffness, which facilitates force transmission for the push-pull actuation of the bending segment 302. The polymer jacket 354 covers the braided wire 352 and the flexible tube 332. The jacket 354 can be constructed of a material configured to cooperate with the material of adjacent structures the insertion shaft engages. For the insertion shaft 320 fixed to the outer tube 306, the jacket 354 can engage a delivery mechanism with which it interfaces, such as a flexible endoscope, to promote low-friction so that the steerable instrument 300 can move freely therein. For the insertion shaft fixed to the inner tube 304, the jacket 354 can engage the flexible tube 332 of the insertion shaft fixed to the outer tube 306, to promote low-friction so that the inner insertion shaft can move freely in the outer insertion shaft, which helps avoid torsional windup and snapping.

At the transition section 340, the insertion shaft 320 has the same basic configuration of the proximal section 350, i.e., it includes the flexible tube 332, braided wire 352, and polymer jacket 354. One difference between the proximal section 350 and the transition section 340 can be that the braided wire 352 of the transition section can be more finely braided (i.e., a high braid density) in comparison to that of the proximal section, which produces higher flexural compliance, torsional stiffness, and kink resistance.

At the transition section 340, the insertion shaft 320 can have a lower flexible stiffness, as this section of the tube is expected to reside within the active bending section of the delivery device, e.g., steerable endoscope, through which it is being delivered, and therefore must be capable of undergoing a smaller radius of curvature than the rest of the insertion shaft. This can be achieved either through a higher density of braid reinforcement 352 (as shown), or through the usage of a low-durometer jacket layer 354 material.

As there is an inverse correlation between flexibility and axial/torsional rigidity, it is advantageous for the proximal section 350 to be axially and torsionally stiff to enable the delivery of axial and torsional forces from the proximal end to the distal end. To achieve this characteristic, the proximal section 350 can be configured to have a higher axial and torsional stiffness, and lower bending compliance, over a much longer length of the tube that is configured to be disposed within the passive section of the endoscope. This can be achieved through a lower density of braid reinforcement 352and/or through the use of a stiffer (higher-durometer) jacket layer 354 material.

The materials used to construct the components of the insertion shaft 320 of the steerable instrument 300 can vary. In one particular configuration of the insertion shaft 320, the flexible tube 332 can constructed of a PTFE material, the braided wire 352 can be stainless steel, the polymer jacket 354 can be constructed of a polyamide material, and the jacket 334 can be a thin layer of the PTFE tube material or a PEBA material connected to the PTFE tube, *e.g., via* an adhesive.

### Methods of Fixation

In one example configuration of the steerable instrument 300, the inner and outer tubes 304, 306 of the bending segment 302 can be attached to their respective insertion shafts 302, i.e., to their respective flexible tubes 332 through an overlapping adhesive bending segment in which the flexible tubes are configured to fit over end portions of the inner/outer tubes and secured with a biocompatible adhesive. For superior adhesion, the polymeric surface being adhered to can be treated at the microstructural level *(e.g., via* plasma etching) to increase surface activation (especially for materials consisting of fluoropolymers, such as PTFE), and any metallic surfaces be cleaned of surface contaminates and roughened.

As this overlapping bending segment will implicitly add wall thickness to the overall tube combination, it is possible to selectively laser-ablate the polymer jacket layer 354 prior to adhesion, thereby reducing the outer diameter of the insertion shaft 332 at the overlap location to reduce the total overlapping bending segment thickness. For an insertion shaft 332 with braided wires 352, it is also possible to extend the jacket layer 354 distally past the point of braid/liner termination (a secondary manufacturing process called 'catheter tipping') to either partially or fully encapsulate the bending segment 302. This is described further herein under the heading "Encapsulation."

### Laser-patterned Nitinol insertion shafts

Instead of forming insertion shafts with separate components - flexible polymer tubes, braided wire, and jacket, the tubes forming the bending segment can extend the entire length of the steerable instrument, and the insertion shafts can be formed *via* laser cut slot patterns formed in the sidewalls thereof. Unlike the slots forming the serpentine beam elements of the bending segment(s), the slots forming the insertion shaft section are configured to exhibit omnidirectional flexural compliance properties required to facilitate delivery through a flexible delivery device, such as a flexible endoscope.

Fig. 18 illustrates an insertion shaft portion 360 formed in a tube 362, which forms a portion of a steerable instrument. The tube 362 can be an outer tube or an inner tube of the steerable instrument having any of the bending segment configurations disclosed herein. Only the insertion shaft 360 portion is shown in Fig. 18. It should be understood that the insertion shaft portion 360 is but one section of the tube and that, implemented in a steerable instrument, a bending segment would be positioned distally of the insertion shaft portion, i.e., to the left of the insertion shaft portion as viewed in Fig. 18.

The insertion shaft portion 360 is formed *via* a repeating pattern of slots 368 that alternates rotationally along the length of the insertion shaft. A distal section 364 of the insertion shaft portion 360 has the slots 368 arranged with a small pitch, which produces flexibility in that portion. The slot pitch increases toward a proximal section 366, which has a reduced degree of flexibility. The laser cut slot pattern of the insertion shaft portion 366 is a circumferential brickwork pattern that serves to reduce the stiffness of the tube while maintaining a neutral axis at the geometric center of the tube, such that the tube is configured to bend omni-directionally, with no singular preferred direction. Alternative patterns, such as interrupted spirals, cell patterns, or interlocking patterns or puzzle piece patterns can also be implemented.

### Eliminating Axial-Torsional Coupling

Axial-torsional coupling occurs when a pure tensile or compressive load placed on a laser-patterned Nitinol shaft induces a twist in the shaft. This often happens when the tube is patterned with an 'interrupted spiral' pattern, where the tube is cut in a spiral fashion with a handedness that wraps around the tube, or is otherwise patterned in a way such that the repeating slot pattern 'twists' around the tube. High axial-torsional coupling in the insertion shaft is deleterious to the overall function of the nested serpentine beam tubes that form the bending segment. In order to function correctly, the high stiffness spines of the serpentine beam patterned tubes should remain in diametric opposition, and any relative twist induced by the insertion shafts when differential forces are applied can cause the backbones to come out of alignment.

To avoid this, cut patterns used in the design of the insertion shaft should eliminate any axial/torsional coupling. This can be done by choosing only patterns with perpendicular cuts, so that any handedness is removed. This can also be done through careful design of the handed patterns such that any relative twist between the two tubes is cancelled out through equal and opposite axial/torsional coupling, by very carefully matching the chiral nature of the insertion shafts that form the inner and outer tube. In other words, if the two tubes comprising the insertion shafts are configured to twist the exact same amount given an equal and opposite tensile force, the torsional coupling effect is nullified and the spines of the serpentine section will remain aligned, permitting bending.

### Variable Insertion Shaft Stiffness:

The laser-cut patterns making up the insertion shaft portion could also be configured to vary along the length of the tube, such that the tube exhibits variable stiffness properties. This is shown in Fig. 19, which shows a flat/planar plan view of a tube 370 with slot patterns that define three sections of an insertion shaft. The tube 370 can be an outer tube or an inner tube of a steerable instrument having any of the bending segment configurations disclosed herein. Fig. 19 illustrates the tube 370 as if it was cut longitudinally and flattened in order to make clear the slot patterns of the tube. As shown in Fig. 19, the tube 370 includes a flexible section 372, a transition section 374, and a stiff section 376 of the insertion shaft, all of which are defined by slots 378 that are laser-cut through the tube sidewall.

The flexible section 372 is configured exhibit axially and torsionally stiff so as to transmit axial and torsional force to the bending segment (not shown). The flexible section 372 is also configured to exhibit bending compliance so that it can conform easily to an active bending section of a delivery device, such as a flexible endoscope.

The stiff section 376 of the tube 370 is significantly longer than the flexible section 372 and transition section 374, and forms the majority of the insertion shaft, as it is configured to extend through the passive portion of the delivery device. As there is an inverse correlation between flexibility and axial/torsional rigidity, it is advantageous for the majority of the insertion shaft, i.e., the stiff section 376, to be as axially and torsionally stiff as possible in order to enable the transmission of axial forces and torques from the proximal end to the distal end, and to limit the amount of tube stretch that occurs due to the application of the differential actuation forces.

The transition section 374 is configured to produce a gradual transition from the flexible section 372 to the stiff section 346, and *vice versa.* Portions of the transition section 374 could also reside within the active bending section of the delivery device, so some bending compliance can prove beneficial.

The characteristics of the sections 372, 374, 376 are configured through the pattern of the slots 378 in the tube 370. In the configuration illustrated in Fig. 19, the slots 378 are configured in a perpendicular cell pattern, which is identical throughout the length of the insertion shaft. The stiffness of the tube is determined by the pitch distance between the slots. In the flexible section 372, the pitch of the slots 378 is small and the slots are uniformly closely spaced. In the stiff section 376, the pitch of the slots 378 is large and the slots are uniformly spaced further apart. In the transition section 374, the pitch varies, increasing gradually from the flexible section 372 to the stiff section 376. The same effect could, however, be achieved through a variation in cut fraction, slot width, total number of slots, or a combination of these parameters.

### Methods for Achieving Multi-Degree-of-Freedom Bending

For any given steerable instrument with a set of n concentric tube pairs having a serpentine beam bending segment, the total number of actuatable degrees of freedom is 3n, since each steerable instrument can be separately and individually inserted/retracted, rotated, and bi-directionally deflected. By nesting one steerable instrument inside the other, the degrees of freedom is doubled, *i.e., n=2,* so 3n=6 degrees of freedom. The degrees of freedom (DoF) can be further increased by nesting additional steerable instruments.

For example, a 6-DoF instrument 400 is illustrated in Figs. 20A-B. In Figs. 20A-B, a first steerable instrument 410 is installed in a second steerable instrument 420. The first steerable instrument 410 includes nested inner and outer tubes 412, 414, that are secured at a distal end of the first steerable instrument. A bending segment 416 of the first steerable instrument is formed with serpentine beam elements according to the example configurations shown and described herein. The bending segment 416 is actuatable *via* push-pull forces applied to the inner and outer tubes 412, 414. The second steerable instrument 420 includes nested inner and outer tubes 422, 424, that are secured at a distal end of the second steerable instrument. A bending segment 426 of the second steerable instrument 420 is formed with serpentine beam elements according to the example configurations shown and described herein. The bending segment 426 is actuatable *via* push-pull forces applied to the inner and outer tubes 422, 424. It is also worth noting here that the first steerable instrument 410 and/or the second steerable instrument 420 can be fitted with respective insertion shaft portions 418, 428 that include the slot features described above with reference to Figs. 18 and 19.

Each of the first and second steerable instruments 410, 420 are actuatable in their respective 3-DoF manner, i.e., insertion/retraction, rotation, and deflection/bending. As shown in Fig. 20B, the outer, second steerable instrument 420 is considered the 'parent' actuator, and the inner, first steerable instrument 410 is considered the 'child.' This is because the child steerable instrument 410 motion is inherently coupled to the motion of the parent steerable instrument 420, but the child can move independently about an inertial frame determined by the parent orientation. As shown in Fig. 20B, the bending segment 426 of the parent steerable instrument 420 is actuated to create a bend, which the child steerable instrument 410 follows, which controls the orientation of the child steerable instrument as it exits the parent. The exit orientation of the child steerable instrument 410 can also be controlled by the other two degrees-of-freedom of the parent steerable instrument 420, i.e., insertion/retraction and rotation. This allows the child steerable instrument 410 to be controlled with its own three degrees-of-freedom from the exit orientation. The instrument 400 therefore enjoys 6-DoF functionality.

In the design of the child steerable instrument 410, it is important that the insertion shaft 418 be flexible enough to enable the full expected range of bending of the bending segment 426 of the parent steerable instrument 420, while being sufficiently stiff, both axially and torsionally, to transmit rotations and forces to the distal end of the child bending segment 416. The child insertion shaft 418 must also be omnidirectionally compliant to eliminate any mechanical bias or preferential bending axes which might also limit the range of bending of the parent steerable instrument 420. This can be achieved, for example, by constructing the child insertion shaft 418 in accordance with the configurations described herein with reference to Figs. 18 and 19. In the example configuration of Figs. 20A-B, the steerable instruments 410, 420 have been configured with a laser-cut Nitinol insertion shafts 418, 428 with brickwork elements that create omnidirectional compliance. This same logic can be extended to an instrument including any number of steerable instruments nested concentrically inside each other to form a multi-degree-of-freedom system.

The parent/child instrument 400 of Figs. 20A-B can be implemented in a variety of manners. For example, referring to Fig. 21A, the 6-DoF instrument 400 can be configured so that the child sheath, i.e., the outer tube 414, can itself be configured to carry a surgical tool, such as a laser fiber 430.

Similarly, a multi-armed system can be created by nesting multiple child steerable instruments 410 in a single parent steerable instrument 420 so that each child is enjoys six degrees-of-freedom, three of which are the same, as they are housed within the same parent. In this instance, the parent steerable instrument 420 can be another steerable instrument (see, Fig. 21B) or flexible endoscope (see, Fig. 21C). In these example configurations, each child steerable instrument 410 can be configured to carry a surgical tool 432, as shown.

### Methods for Stress Concentration Reduction

Material stress is known to concentrate and increase around sharp or sudden discontinuities in material. These stress concentrations can lead to material failure if not properly accounted for. Fig. 22 illustrates a portion of a tube 450 configured to form a portion of a bending segment 452 with a serpentine beam element 454 defined by a plurality of slots 456 cut through the tube sidewall. To reduce stress concentrations that might form at the end or root of each slot 456, the base of each slot can include a stress relieving cut-out 458 that extends the root axially, in the direction of the central axis of the tube 450. In the example configuration of Fig. 22, the cut-outs 458 resemble a T-shape. Other shapes, such as circular or elliptical cutouts, can also be used. In extending the roots of the slots 456 axially, the surface area over which bending stresses can resolve is increased, and stress concentrations are reduced at the slot roots where, as described above, stress concentrations might otherwise occur.

### Encapsulation

The encapsulation of the Nitinol tubes used to construct the steerable instruments described herein, such as where the instrument forms a portion of an instrument that includes an insertion shaft (see, Fig. 17), can be implemented in a variety of manners. For example, the Nitinol tubes that form the steerable instruments described herein can be disposed within another low-durometer biocompatible material (e.g., PEBAX 35D^{®}, a low-durometer PEBA material available commercially from Arkema, Inc.) to encapsulate and make watertight the slots, and also to prevent tissue from being 'pinched' within the slots during normal operation. This encapsulation layer can also serve the purpose of electrically insulating the Nitinol tip which may be important in clinical applications that require the use of electrosurgery. This encapsulation layer can have radio-opaque features to improve the visibility of the steerable tip under medical imaging. The encapsulating material must be carefully selected such that its flexural stiffness does not detract significantly from the bending capabilities of the steerable instrument.

As another alternative, "pinching" can be prevented by making the slot width so small/narrow that tissue cannot enter the space. In fact, slow widths can be made so small/narrow that even liquids wont pass through them. Therefore, this narrow slot width bending segment configuration could be used to prevent tissue pinching and also to make the bending segment watertight.

Similarly, a liner material may be disposed within the inner-diameter of the steerable tube to enhance lubricity and promote the passage of tools. As before, this liner material must be chosen carefully such that it does not prohibit the bending of the steerable tip.

### Application Example: A Bimanual Steerable instrument for Flexible Endoscopy

In an example application that combines multiple example configurations described herein, a combination of various serpentine beam steerable instruments can be configured to create a bimanually dexterous manipulation system 460 delivered through a dual-channel flexible endoscope 462 with illumination LEDs 464 and a camera 466. This is shown in Fig. 23. As shown in Fig. 23, a flexible endoscope 462, is outfitted with a pair of 6-DoF steerable instruments 470, 480. The first steerable instrument 470 includes a child steerable instrument 472 that is nested within a parent steerable instrument 474 and carries a surgical tool 476. The second steerable instrument 480 also includes a child steerable instrument 482 that is nested within a parent steerable instrument 484 and carries a surgical tool 486. The endoscope 460, being flexible and actuatable, lends another three degrees-of-freedom to the system 460, i.e., to each of the steerable instruments 470, 480.

As can be seen in Fig. 23, the parent steerable instruments 474, 484 include dual serpentine beam bending segments, such as those described herein with reference to Figs. 14A-C. When actuated, parent steerable instruments 474, 484 adopt an S-shape, and self-collision of the slots (see, Fig. 4) increases their stiffnesses to provide a stable platform. The child steerable instruments 472, 482 pass through their respective parent steerable instruments 474, 484, and are each independently actuatable 3-DoF steerable instruments (roll, insertion/retraction, and deflection/bending). The child steerable instruments 472, 482 deliver the tools 476 for interacting with tissue, such as a forceps and an electrosurgical probe, as shown. The system/platform 460 of Fig. 23 can be used in a variety of procedures, such as gastrointestinal endoscopic procedures that require simultaneous retraction and cutting of tissue, e.g., Endoscopic Submucosal Dissection (ESD).

## Claims

1. A surgical system (400, 460) comprising:
a steerable instrument (20, 220) comprising:
a first tube (22, 230) comprising a first serpentine beam (30, 234) formed in a tubular sidewall thereof;
a second tube (24, 240) comprising a second serpentine beam (32, 244) formed in a tubular sidewall thereof;
wherein the first and second tubes (22, 230, 24, 240) are concentrically nested and positioned so that the first and second serpentine beams (30, 234, 32, 244) are at least partially aligned with each other axially and face in radial directions that differ angularly from one another;
wherein the first and second tubes (22, 230, 24, 240) are connected to each other distally of the first and second serpentine beams (30, 234, 32, 244);
wherein the first tube (22, 230) comprises an uncut spine extending longitudinally along the first tube (22, 230), the first serpentine beam (30, 234) extending longitudinally along the first tube (22, 230) opposite the uncut spine;
wherein the first and second serpentine beams (30, 234, 32, 244) define a bending segment (40, 222) that is actuatable to form a bend in the nested tube structure in response to differential axial forces applied to the first and second tubes (22, 230, 24, 240).

2. The surgical system (400, 460) recited in claim 1, wherein:
the first tube (22, 230) comprises a plurality of parallel first slots (26, 236) that extend through the sidewall of the first tube (22, 230) perpendicular to a central axis of the first tube (22, 230), the first slots (26, 236) being offset angularly in a back-and-forth manner, wherein the first slots (26, 236) define the first serpentine beam (30, 234); and
the second tube (24, 240) comprises a plurality of parallel second slots (28, 246) that extend through the sidewall of the second tube (24, 240) perpendicular to a central axis of the second tube (24, 246), the second slots (28, 246) being offset angularly in a back-and-forth manner, wherein the second slots (28, 246) define the second serpentine beam (32, 244).

3. The surgical system (400, 460) recited in claim 2, wherein:
the first serpentine beam (30, 234) comprises lateral beam portions (54a) that extend parallel to each other on opposite sides of the first slots (26, 236), and longitudinal beam portions (54b) that interconnect adjacent ends of the lateral beam portions (54a) in an alternating manner; and
the second serpentine beam (32, 244) comprises lateral beam portions (54a) that extend parallel to each other on opposite sides of the second slots (28, 246), and longitudinal beam portions (54b) that interconnect adjacent ends of the lateral beam (54a) portions in an alternating manner.

4. The surgical system (400, 460) recited in any one of claims 1 to 3, wherein the first and second serpentine beams (30, 234, 32, 244) are configured so that adjacent lateral beam portions (54a) of the first and second serpentine beams (30, 234, 32, 244) engage each other at touch points (58) that limit deflection of the bending segment (40, 222),
optionally wherein the touch points (58) are configured to define a fully deflected condition of the bending segment (40, 222),
further optionally wherein the bending segment (40, 222) is configured so that the differential axial forces applied to the first and second tubes (22, 230, 24, 240), urge the touch points (58) against each other to increase the stiffness of the bending segment (40, 222) in the fully deflected condition.

5. The surgical system (400, 460) recited in any one of claims 1 to 4, wherein the first tube (22, 230) comprises a plurality of first serpentine beams (30, 234) that extend parallel to each other along the length of the bending segment (40, 222) and the second tube (24, 240) comprises a plurality of second serpentine beams (32, 244) that extend parallel to each other along the length of the bending segment (40, 222),
optionally wherein:
the first tube (22, 230) comprises a plurality of parallel first slots (26, 236) that extend through the sidewall of the first tube (22, 230) perpendicular to a central axis of the first tube (22, 230) , wherein the first slots (26, 236) are arranged in rows of plural slots spaced radially from each other, and wherein each row of first slots (26, 236) is offset angularly from adjacent rows, wherein the first slots (26, 236) define the plurality of first serpentine beams (30, 234); and
the second tube (24, 240) comprises a plurality of parallel second slots (28, 246) that extend through the sidewall of the second tube (24, 240) perpendicular to a central axis of the second tube (24, 240), wherein the second slots (28, 246) are arranged in rows of plural slots spaced radially from each other, and wherein each row of second slots (28, 246) is offset angularly from adjacent rows, wherein the second slots (28, 246) define the plurality of second serpentine beams (32, 244),
further optionally wherein the first slots (26, 236) in each row are equal in length and the second slots (28, 246) in each row are equal in length, or wherein the first slots (26, 236) in each row are of two different lengths arranged in an alternating fashion, and wherein the second slots (28. 236) in each row are of two different lengths arranged in an alternating fashion.

6. The surgical system (400, 460) recited in any one of claims 1 to 5, wherein the steerable instrument (20, 220) comprises a plurality of bending segments (40, 222) spaced axially along the length of the steerable instrument (20, 220), wherein plurality of bending segments (20, 220) comprise a first bending segment (40, 222) and a second bending segment (40, 222), the first and second bending segments (40, 222) each being formed by respective first serpentine beams (30, 234) formed in the first tube (22, 230) sidewall and second serpentine beams (32, 244) formed in the second tube (24, 240) sidewall that are axially aligned with each other, optionally wherein the first and second bending segments (40, 222) are configured to deflect in different directions when actuated; or,
wherein the first and second bending segments (40, 222) are separated by transition regions of the first and second tubes (22, 230, 24, 240) that are free from slots in the tube sidewalls; or,
wherein the first and second bending segments (40, 222) are configured to deflect in opposite directions so that the steerable instrument (20, 220) assumes an S-shape when actuated.

7. The surgical system (400, 460) recited in any one of claims 1 to 5,
wherein the angular positions of the first and second slots (26, 236, 28, 248) are adjusted progressively about the central axis so that the bending segment (40, 222) bends out of plane in a helical manner when actuated.

8. The surgical system (400, 460) recited in any of claims 2 to 7, wherein the first and second slots (26, 236, 28, 246) comprise contact aids that limit bending motion of the bending segment (40, 222) in opposite bending directions,
optionally wherein each contact aid comprises a convexly shaped member protruding from a lateral beam member positioned in a correspondingly shaped concave receiver on an adjacent lateral beam member (54a); or,
wherein each contact aid has a dovetail configuration wherein the convexly shaped member comprises a dovetail pin and the concave receiver comprises a dovetail tail.

9. The surgical system (400, 460) recited in any one of claims 1 to 8, wherein the steerable instrument (20, 220) comprises a first insertion shaft (320) extending proximally from the first serpentine beam (30, 234) and a second insertion shaft (320) extending proximally from the second serpentine beam (32, 244), the first and second insertion shafts (320) being configured to be flexurally compliant to allow them to adopt to a tortuous path, omnidirectionally compliant so that they exhibit the same flexural stiffness regardless of the direction of loading, with axial stiffness and torsional rigidity to sufficiently transmit forces and torques from proximal ends (310) of the insertion shafts (320) to the distally located bending segment (40, 222) of the steerable instrument (20, 220).

10. The surgical system (400, 460) recited in claim 9, wherein:
the first insertion shaft (320) comprises a polymeric flexible tube (332) positioned adjacent and connected to the first tube (22, 230), a braided wire reinforcement layer (352) overlying or embedded in the flexible tube (332), and polymer jacket (354) that covers the braided wire (352) and helps secure the braided wire (352) on the flexible tube (332); and
the second insertion shaft (320) comprises a polymeric flexible tube (332) positioned adjacent and connected to the second tube (24, 240), a braided wire reinforcement layer (352) overlying or embedded in the flexible tube (332), and polymer jacket (354) that covers the braided wire (352) and helps secure the braided wire (352) on the flexible tube (332),
optionally wherein the braid density of the braided wire (352) is varied along the length of the at least one of the first and second insertion shafts (320) to adjust the flexibility, axial stiffness, and torsional rigidity of the insertion shaft (320) along its length; and,,
further optionally wherein the flexible tubes (332) are secured to their respective second tubes (24, 240) by one of embedding the tube (24, 240) in the flexible tube (332) material, connecting the flexible tube (332) to the outer tube (354) with an adhesive, and forming the flexible tube (332) as a thin jacket of material adhered to the outer tube (354).

11. The surgical system (400, 460) recited in claim 9, wherein the first and second insertion shafts (320) comprise respective portions of the first and second tubes (22, 230, 24, 240) extending proximally of their respective serpentine beams (30, 234, 32, 244) and comprising a plurality of slots (26, 236, 28, 246) in their respective tube sidewalls, the slots (26, 236, 28, 246) extending perpendicular to the central axis of the first and second tubes (22, 230, 24, 240) and being arranged in rows that extend radially about the central axis and are configured so that the first and second insertion shafts (320) are flexurally compliant to allow them to adopt to a tortuous path, omnidirectionally compliant so that they exhibits the same flexural stiffness regardless of the direction of loading, and axial stiff and torsionally rigid so that they transmit forces and torques from the proximal ends of the insertion shafts (320) to the distally located bending segment of the steerable instrument (20, 220),
optionally wherein the pitch of the slots (26, 236, 28, 246) arranged along the first and second insertion shafts (320) are varied along the lengths of the first and second insertion shafts (320) to adjust the flexibility, axial stiffness, and torsional rigidity of the first and second insertion shafts (320) along their lengths.

12. The surgical system (400, 460) recited in claim 2, wherein the steerable instrument (20, 220) further comprises a polymer jacket (354) that covers the bending segment (40, 222) and prevents tissue from entering the slots (26, 236, 28, 246); or,
wherein the slots (26, 236, 28, 246) are configured to be so narrow that tissue cannot enter the slots (26, 236, 28, 246).

13. The surgical system (400, 460) recited in any of the preceding claims, wherein the steerable instrument (20, 220) comprises a compound steerable instrument comprising first and second steerable instruments (410, 420), wherein the second steerable instrument (420) is configured to extend through an inner lumen of the first steerable instrument (410).

14. The surgical system (400, 460) recited in any of the preceding claims, further comprising a delivery device configured to be inserted and manipulated to position at a surgical site, wherein the steerable instrument (20, 220) is configured to be advanced through the delivery device and to have the bending segment (40, 222) positioned outside the delivery device with the remainder of the steerable instrument (20, 220) positioned in and supported by the delivery device,
optionally wherein the delivery device comprises one of an endoscope, a flexible endoscope, and another steerable instrument.

15. The surgical system (400, 460) recited in claim 14, wherein the delivery device is configured so that the steerable instrument (20, 220), supported by the delivery device with the bending segment (40, 222) positioned outside the delivery device, can support a surgical instrument extending through the inner lumen of the nested tube structure for tissue manipulation at the surgical site.

## Patentansprüche

1. Chirurgisches System (400, 460), umfassend:
ein steuerbares Instrument (20, 220), umfassend:
ein erstes Rohr (22, 230), umfassend einen ersten serpentinenförmigen Träger (30, 234), der in einer rohrförmigen Seitenwand davon gebildet ist;
ein zweites Rohr (24, 240), umfassend einen zweiten serpentinenförmigen Träger (32, 244), der in einer rohrförmigen Seitenwand davon gebildet ist;
wobei das erste und das zweite Rohr (22, 230, 24, 240) konzentrisch ineinander verschachtelt und so positioniert sind, dass der erste und der zweite serpentinenförmige Träger (30, 234, 32, 244) mindestens teilweise axial miteinander ausgerichtet sind und in radialen Richtungen einander zugewandt sind, die sich winkelmäßig voneinander unterscheiden;
wobei das erste und das zweite Rohr (22, 230, 24, 240) distal von dem ersten und dem zweiten serpentinenförmigen Träger (30, 234, 32, 244) miteinander verbunden sind;
wobei das erste Rohr (22, 230) einen ungeschnittenen, sich in Längsrichtung entlang des ersten Rohrs (22, 230) erstreckenden Grat umfasst, wobei sich der erste serpentinenförmige Träger (30, 234) in Längsrichtung entlang des ersten Rohrs (22, 230) gegenüber dem ungeschnittenen Grat erstreckt;
wobei der erste und der zweite serpentinenförmige Träger (30, 234, 32, 244) ein Biegesegment (40, 222) definieren, das betätigbar ist, um als Reaktion auf unterschiedliche axiale Kräfte, die auf das erste und das zweite Rohr (22, 230, 24, 240) angewendet werden, eine Biegung in der verschachtelten Rohrstruktur zu bilden.

2. Chirurgisches System (400, 460) nach Anspruch 1, wobei:
das erste Rohr (22, 230) eine Vielzahl paralleler erster Schlitze (26, 236) umfasst, die sich durch die Seitenwand des ersten Rohrs (22, 230) senkrecht zu einer Mittelachse des ersten Rohrs (22, 230) hindurch erstrecken, wobei die ersten Schlitze (26, 236) winkelmäßig auf eine Hin-und-her-Weise versetzt sind, wobei die ersten Schlitze (26, 236) den ersten serpentinenförmigen Träger (30, 234) definieren; und
das zweite Rohr (24, 240) eine Vielzahl paralleler zweiter Schlitze (28, 246) umfasst, die sich durch die Seitenwand des zweiten Rohrs (24, 240) senkrecht zu einer Mittelachse des zweiten Rohrs (24, 246) hindurch erstrecken, wobei die zweiten Schlitze (28, 246) winkelmäßig auf eine Hin-und-her-Weise versetzt sind, wobei die zweiten Schlitze (28, 246) den zweiten serpentinenförmigen Träger (32, 244) definieren.

3. Chirurgisches System (400, 460) nach Anspruch 2, wobei:
der erste serpentinenförmige Träger (30, 234) seitliche Trägerabschnitte (54a), die sich parallel zueinander auf gegenüberliegenden Seiten der ersten Schlitze (26, 236) erstrecken, und Längsträgerabschnitte (54b) umfasst, die benachbarte Enden der seitlichen Trägerabschnitte (54a) auf eine abwechselnde Weise miteinander verbinden; und
der zweite serpentinenförmige Träger (32, 244) seitliche Trägerabschnitte (54a), die sich parallel zueinander auf gegenüberliegenden Seiten der zweiten Schlitze (28, 246) erstrecken, und Längsträgerabschnitte (54b) umfasst, die benachbarte Enden der seitlichen Trägerabschnitte (54a) auf eine abwechselnde Weise miteinander verbinden.

4. Chirurgisches System (400, 460) nach einem der Ansprüche 1 bis 3, wobei der erste und der zweite serpentinenförmige Träger (30, 234, 32, 244) so konfiguriert sind, dass benachbarte seitliche Trägerabschnitte (54a) des ersten und des zweiten serpentinenförmigen Trägers (30, 234, 32, 244) an Berührungspunkten (58) miteinander in Eingriff kommen, die die Auslenkung des Biegesegments (40, 222) begrenzen,
wobei die Berührungspunkte (58) optional konfiguriert sind, um einen vollständig ausgelenkten Zustand des Biegesegments (40, 222) zu definieren,
wobei das Biegesegment (40, 222) weiter optional so konfiguriert ist, dass die auf das erste und das zweite Rohr (22, 230, 24, 240) angewendeten differentiellen Axialkräfte die Berührungspunkte (58) gegeneinander drücken, um die Steifigkeit des Biegesegments (40, 222) in dem vollständig ausgelenkten Zustand zu erhöhen.

5. Chirurgisches System (400, 460) nach einem der Ansprüche 1 bis 4, wobei das erste Rohr (22, 230) eine Vielzahl erster serpentinenförmiger Träger (30, 234) umfasst, die sich entlang der Länge des Biegesegments (40, 222) parallel zueinander erstrecken, und das zweite Rohr (24, 240) eine Vielzahl zweiter serpentinenförmiger Träger (32, 244) umfasst, die sich entlang der Länge des Biegesegments (40, 222) parallel zueinander erstrecken,
wobei optional:
das erste Rohr (22, 230) eine Vielzahl paralleler erster Schlitze (26, 236) umfasst, die sich durch die Seitenwand des ersten Rohrs (22, 230) senkrecht zu einer Mittelachse des ersten Rohrs (22, 230) hindurch erstrecken, wobei die ersten Schlitze (26, 236) in Reihen von mehreren, radial voneinander beabstandeten Schlitzen angeordnet sind und wobei jede Reihe von ersten Schlitzen (26, 236) von benachbarten Reihen winkelmäßig versetzt ist, wobei die ersten Schlitze (26, 236) die Vielzahl erster serpentinenförmiger Träger (30, 234) definieren; und
das zweite Rohr (24, 240) eine Vielzahl paralleler zweiter Schlitze (28, 246) umfasst, die sich durch die Seitenwand des zweiten Rohrs (24, 240) senkrecht zu einer Mittelachse des zweiten Rohrs (24, 240) hindurch erstrecken, wobei die zweiten Schlitze (28, 246) in Reihen von mehreren, radial voneinander beabstandeten Schlitzen angeordnet sind, und wobei jede Reihe von zweiten Schlitzen (28, 246) von benachbarten Reihen winkelmäßig versetzt ist, wobei die zweiten Schlitze (28, 246) die Vielzahl zweiter serpentinenförmiger Träger (32, 244) definieren,
wobei die ersten Schlitze (26, 236) weiter optional in jeder Reihe in der Länge gleich sind und die zweiten Schlitze (28, 246) in jeder Reihe in der Länge gleich sind, oder wobei die ersten Schlitze (26, 236) in jeder Reihe von zwei unterschiedlichen Längen sind, die auf eine abwechselnde Art und Weise angeordnet sind, und wobei die zweiten Schlitze (28, 236) in jeder Reihe von zwei unterschiedlichen Längen sind, die auf eine abwechselnde Art und Weise angeordnet sind.

6. Chirurgisches System (400, 460) nach einem der Ansprüche 1 bis 5, wobei das steuerbare Instrument (20, 220) eine Vielzahl von Biegesegmenten (40, 222) umfasst, die axial entlang der Länge des steuerbaren Instruments (20, 220) beabstandet sind, wobei die Vielzahl von Biegesegmenten (20, 220) ein erstes Biegesegment (40, 222) und ein zweites Biegesegment (40, 222) umfasst, wobei das erste und das zweite Biegesegment (40, 222) jeweils von jeweiligen ersten serpentinenförmigen Trägern (30, 234), die in der Seitenwand des ersten Rohrs (22, 230) gebildet sind, und zweiten serpentinenförmigen Trägern (32, 244), die in der Seitenwand des zweiten Rohrs (24, 240) gebildet sind, gebildet werden, die axial miteinander ausgerichtet sind, wobei das erste und das zweite Biegesegment (40, 222) optional konfiguriert sind, um bei Betätigung in unterschiedliche Richtungen auszulenken; oder,
wobei das erste und das zweite Biegesegment (40, 222) durch Übergangsbereiche des ersten und des zweiten Rohrs (22, 230, 24, 240) getrennt sind, die frei von Schlitzen in den Rohrseitenwänden sind; oder
wobei das erste und das zweite Biegesegment (40, 222) konfiguriert sind, um so in entgegengesetzte Richtungen auszulenken, dass das steuerbare Instrument (20, 220) bei Betätigung eine S-Form annimmt.

7. Chirurgisches System (400, 460) nach einem der Ansprüche 1 bis 5, wobei die Winkelpositionen der ersten und der zweiten Schlitze (26, 236, 28, 248) progressiv um die Mittelachse so eingestellt werden, dass sich das Biegesegment (40, 222) bei Betätigung auf spiralförmige Weise aus der Ebene biegt.

8. Chirurgisches System (400, 460) nach einem der Ansprüche 2 bis 7, wobei die ersten und die zweiten Schlitze (26, 236, 28, 246) Kontakthilfsmittel umfassen, die die Biegebewegung des Biegesegments (40, 222) in entgegengesetzten Biegerichtungen begrenzen,
wobei jedes Kontakthilfsmittel optional ein konvex geformtes Element umfasst, das aus einem seitlichen Trägerelement herausragt, das in einer entsprechend geformten konkaven Aufnahme auf einem benachbarten seitlichen Trägerelement positioniert ist (54a); oder
wobei jedes Kontakthilfsmittel eine Schwalbenschwanzkonfiguration aufweist, wobei das konvex geformte Element einen Schwalbenschwanzstift umfasst und die konkave Aufnahme einen Schwalbenschwanzschwanz umfasst.

9. Chirurgisches System (400, 460) nach einem der Ansprüche 1 bis 8, wobei das steuerbare Instrument (20, 220) einen ersten Einführungsschaft (320), der sich proximal von dem ersten serpentinenförmigen Träger (30, 234) erstreckt, und einen zweiten Einführungsschaft (320) umfasst, der sich proximal von dem zweiten serpentinenförmigen Träger (32, 244) erstreckt, wobei der erste und der zweite Einführungsschaft (320) konfiguriert sind, um biegbar nachgiebig zu sein, um ihnen zu erlauben, einen gewundenen Pfad omnidirektional nachgiebig so anzunehmen, dass sie unabhängig von der Belastungsrichtung die gleiche Biegesteifigkeit mit axialer Steifigkeit und Torsionsstarrheit vorweisen, um Kräfte und Drehmomente von proximalen Enden (310) der Einführungsschäfte (320) auf das distal gelegene Biegesegment (40, 222) des steuerbaren Instruments (20, 220) ausreichend zu übertragen.

10. Chirurgisches System (400, 460) nach Anspruch 9, wobei:
der erste Einführungsschaft (320) ein flexibles Polymerrohr (332), das dem ersten Rohr (22, 230) benachbart positioniert und mit diesem verbunden ist, eine geflochtene Drahtverstärkungsschicht (352), die das flexible Rohr (332) überlagert oder in dieses eingebettet ist, und einen Polymermantel (354) umfasst, der den geflochtenen Draht (352) bedeckt und dabei hilft, den geflochtenen Draht (352) auf dem flexiblen Rohr (332) zu sichern; und
der zweite Einführungsschaft (320) ein flexibles Polymerrohr (332), das dem zweiten Rohr (24, 240) benachbart positioniert und mit diesem verbunden ist, eine geflochtene Drahtverstärkungsschicht (352), die das flexible Rohr (332) überlagert oder in dieses eingebettet ist, und einen Polymermantel (354) umfasst, der den geflochtenen Draht (352) bedeckt und dabei hilft, den geflochtenen Draht (352) auf dem flexiblen Rohr (332) zu sichern,
wobei die Flechtdichte des geflochtenen Drahtes (352) optional entlang der Länge des mindestens einen des ersten und des zweiten Einführungsschafts (320) variiert wird, um die Flexibilität, die axiale Steifigkeit und die Torsionsstarrheit des Einführungsschafts (320) entlang seiner Länge einzustellen; und,
weiter optional, wobei die flexiblen Rohre (332) an ihren jeweiligen zweiten Rohren (24, 240) gesichert werden durch eines von Einbetten des Rohrs (24, 240) in das Material des flexiblen Rohrs (332), Verbinden des flexiblen Rohrs (332) mit dem äußeren Rohr (354) mit einem Klebstoff und Bilden des flexiblen Rohrs (332) als einen dünnen Mantel aus Material, das an dem äußeren Rohr (354) angehaftet ist.

11. Chirurgisches System (400, 460) nach Anspruch 9, wobei der erste und der zweite Einführungsschaft (320) jeweils Abschnitte des ersten und des zweiten Rohrs (22, 230, 24, 240) umfassen, die sich proximal von ihren jeweiligen serpentinenförmigen Trägern (30, 234, 32, 244) erstrecken und eine Vielzahl von Schlitzen (26, 236, 28, 246) in ihren jeweiligen Rohrseitenwänden umfassen, die sich senkrecht zu der Mittelachse des ersten und des zweiten Rohrs (22, 230, 24, 240) erstrecken und in Reihen angeordnet sind, die sich radial um die Mittelachse herum erstrecken und so konfiguriert sind, dass der erste und der zweite Einführungsschaft (320) biegbar nachgiebig sind, um ihnen zu erlauben, einen gewundenen Pfad omnidirektional nachgiebig so anzunehmen, dass sie unabhängig von der Belastungsrichtung die gleiche Biegesteifigkeit aufweisen, sowie axial steif und torsionssteif sind, so dass sie Kräfte und Drehmomente von den proximalen Enden der Einführungsschäfte (320) auf das distal gelegene Biegesegment des steuerbaren Instruments (20, 220) übertragen,
wobei die Teilung der Schlitze (26, 236, 28, 246), die entlang des ersten und des zweiten Einführungsschafts (320) angeordnet sind, optional entlang der Längen des ersten und des zweiten Einführungsschafts (320) variiert werden, um die Flexibilität, die axiale Steifigkeit und die Torsionsstarrheit des ersten und des zweiten Einführungsschafts (320) entlang ihrer Längen einzustellen.

12. Chirurgisches System (400, 460) nach Anspruch 2, wobei das steuerbare Instrument (20, 220) weiter einen Polymermantel (354) umfasst, der das Biegesegment (40, 222) bedeckt und Gewebe daran hindert, in die Schlitze (26, 236, 28, 246) einzutreten; oder
wobei die Schlitze (26, 236, 28, 246) konfiguriert sind, um so schmal zu sein, dass Gewebe nicht in die Schlitze (26, 236, 28, 246) eintreten kann.

13. Chirurgisches System (400, 460) nach einem der vorstehenden Ansprüche, wobei das steuerbare Instrument (20, 220) ein zusammengesetztes steuerbares Instrument umfasst, das ein erstes und ein zweites steuerbares Instrument (410, 420) umfasst, wobei das zweite steuerbare Instrument (420) konfiguriert ist, um sich durch ein inneres Lumen des ersten steuerbaren Instruments (410) hindurch zu erstrecken.

14. Chirurgisches System (400, 460) nach einem der vorstehenden Ansprüche, weiter umfassend eine Abgabevorrichtung, die konfiguriert ist, um an einer Operationsstelle eingeführt und an eine Position manipuliert zu werden, wobei das steuerbare Instrument (20, 220) konfiguriert ist, um durch die Abgabevorrichtung hindurch vorgeschoben zu werden und um das Biegesegment (40, 222) außerhalb der Abgabevorrichtung positioniert aufzuweisen, wobei der Rest des steuerbaren Instruments (20, 220) in der Abgabevorrichtung positioniert ist und von dieser gestützt wird,
wobei die Abgabevorrichtung optional eins von einem Endoskop, einem flexiblen Endoskop und einem anderen steuerbaren Instrument umfasst.

15. Chirurgisches System (400, 460) nach Anspruch 14, wobei die Abgabevorrichtung so konfiguriert ist, dass das von der Abgabevorrichtung getragene steuerbare Instrument (20, 220) mit dem Biegesegment (40, 222) außerhalb der Abgabevorrichtung positioniert ein chirurgisches Instrument tragen kann, das sich durch das innere Lumen der verschachtelten Rohrstruktur hindurch zur Gewebemanipulation an der Operationsstelle erstreckt.

## Revendications

1. Système chirurgical (400, 460) comprenant :
un instrument orientable (20, 220) comprenant :
un premier tube (22, 230) comprenant une première poutre serpentine (30, 234) formée dans une paroi latérale tubulaire de celui-ci ;
un deuxième tube (24, 240) comprenant une deuxième poutre serpentine (32, 244) formée dans une paroi latérale tubulaire de celui-ci ;
dans lequel les premier et deuxième tubes (22, 230, 24, 240) sont emboîtés concentriquement et positionnés de sorte que les première et deuxième poutres serpentines (30, 234, 32, 244) soient au moins partiellement alignées axialement l'une avec l'autre et orientées dans des directions radiales qui diffèrent angulairement l'une de l'autre ;
dans lequel les premier et deuxième tubes (22, 230, 24, 240) sont connectés l'un à l'autre de manière distale par rapport aux première et deuxième poutres serpentines (30, 234, 32, 244) ;
dans lequel le premier tube (22, 230) comprend une colonne non coupée s'étendant longitudinalement le long du premier tube (22, 230), la première poutre serpentine (30, 234) s'étendant longitudinalement le long du premier tube (22, 230) à l'opposé de la colonne non coupée ;
dans lequel les première et deuxième poutres serpentines (30, 234, 32, 244) définissent un segment de flexion (40, 222) qui peut être actionné pour former un coude dans la structure tubulaire imbriquée en réponse à des forces axiales différentielles appliquées aux premier et deuxième tubes (22, 230, 24, 240).

2. Système chirurgical (400, 460) selon la revendication 1, dans lequel :
le premier tube (22, 230) comprend une pluralité de premières fentes parallèles (26, 236) qui s'étendent à travers la paroi latérale du premier tube (22, 230) perpendiculairement à un axe central du premier tube (22, 230), les premières fentes (26, 236) étant décalées de manière angulaire d'avant en arrière, dans lequel les premières fentes (26, 236) définissent la première poutre serpentine (30, 234) ; et
le deuxième tube (24, 240) comprend une pluralité de deuxièmes fentes parallèles (28, 246) qui s'étendent à travers la paroi latérale du deuxième tube (24, 240) perpendiculairement à un axe central du deuxième tube (24, 246), les deuxièmes fentes (28, 246) étant décalées de manière angulaire d'avant en arrière, dans lequel les deuxièmes fentes (28, 246) définissent la deuxième poutre serpentine (32, 244).

3. Système chirurgical (400, 460) selon la revendication 2, dans lequel :
la première poutre serpentine (30, 234) comprend des portions de poutre latérales (54a) qui s'étendent parallèlement l'une à l'autre de part et d'autre des premières fentes (26, 236), et des portions de poutre longitudinales (54b) qui relient des extrémités adjacentes des portions de poutre latérales (54a) de manière alternée ; et
la deuxième poutre serpentine (32, 244) comprend des portions de poutre latérales (54a) qui s'étendent parallèlement l'une à l'autre sur les côtés opposés des deuxièmes fentes (28, 246), et des portions de poutre longitudinales (54b) qui relient les extrémités adjacentes des portions de poutre latérales (54a) de manière alternée.

4. Système chirurgical (400, 460) selon l'une quelconque des revendications 1 à 3, dans lequel les première et deuxième poutres serpentines (30, 234, 32, 244) sont configurées de sorte que les portions latérales adjacentes des poutres (54a) des première et deuxième poutres serpentines (30, 234, 32, 244) se mettent en prise l'une dans l'autre à des points de contact (58) qui limitent la déflexion du segment de flexion (40, 222),
éventuellement dans lequel les points de contact (58) sont configurés pour définir une condition de déformation complète du segment de flexion (40, 222),
en outre, en option, dans lequel le segment de flexion (40, 222) est configuré de sorte que les forces axiales différentielles appliquées aux premier et deuxième tubes (22, 230, 24, 240) poussent les points de contact (58) l'un contre l'autre pour augmenter la rigidité du segment de flexion (40, 222) dans la condition entièrement déformée.

5. Système chirurgical (400, 460) selon l'une quelconque des revendications 1 à 4, dans lequel le premier tube (22, 230) comprend une pluralité de premières poutres serpentines (30, 234) qui s'étendent parallèlement les unes aux autres sur toute la longueur du segment de flexion (40, 222) et le deuxième tube (24, 240) comprend une pluralité de deuxièmes poutres serpentines (32, 244) qui s'étendent parallèlement les unes aux autres sur toute la longueur du segment de flexion (40, 222),
éventuellement dans lequel :
le premier tube (22, 230) comprend une pluralité de premières fentes parallèles (26, 236) qui traversent la paroi latérale du premier tube (22, 230) perpendiculairement à un axe central du premier tube (22, 230), dans lequel les premières fentes (26, 236) sont agencées en rangées de plusieurs fentes espacées radialement les unes des autres, et dans lequel chaque rangée de premières fentes (26, 236) est décalée angulairement par rapport aux rangées adjacentes, dans lequel les premières fentes (26, 236) définissent la pluralité de premières poutres serpentines (30, 234) ; et
le deuxième tube (24, 240) comprend une pluralité de deuxièmes fentes parallèles (28, 246) qui s'étendent à travers la paroi latérale du deuxième tube (24, 240) perpendiculairement à un axe central du deuxième tube (24, 240), dans lequel les deuxièmes fentes (28, 246) sont agencées en rangées de plusieurs fentes espacées radialement les unes des autres, et dans lequel chaque rangée de deuxièmes fentes (28, 246) est décalée angulairement par rapport aux rangées adjacentes, dans lequel les deuxièmes fentes (28, 246) définissent la pluralité de deuxièmes poutres serpentines (32, 244),
en outre, en option, dans lequel les premières fentes (26, 236) de chaque rangée sont de longueur égale et les deuxièmes fentes (28, 246) de chaque rangée sont de longueur égale, ou dans lequel les premières fentes (26, 236) de chaque rangée sont de deux longueurs différentes agencées de manière alternée, et dans lequel les deuxièmes fentes (28, 236) de chaque rangée sont de deux longueurs différentes disposées de manière alternée.

6. Système chirurgical (400, 460) selon l'une quelconque des revendications 1 à 5, dans lequel l'instrument orientable (20, 220) comprend une pluralité de segments de flexion (40, 222) espacés axialement sur toute la longueur de l'instrument orientable (20, 220), dans lequel la pluralité de segments de flexion (20, 220) comprennent un premier segment de flexion (40, 222) et un deuxième segment de flexion (40, 222), les premier et deuxième segments de flexion (40, 222) étant formés respectivement par de premières poutres serpentines (30, 234) formées dans la paroi latérale du premier tube (22, 230) et de deuxièmes poutres serpentines (32, 244) formées dans la paroi latérale du deuxième tube (24, 240), alignées axialement les unes avec les autres, éventuellement dans lequel les premier et deuxième segments de flexion (40, 222) sont configurés pour se déformer dans des directions différentes une fois actionnés ; ou,
dans lequel les premier et deuxième segments de flexion (40, 222) sont séparés par des régions de transition des premier et deuxième tubes (22, 230, 24, 240) qui sont exemptes de fentes dans les parois latérales des tubes ; ou,
dans lequel les premier et deuxième segments de flexion (40, 222) sont configurés pour se déformer dans des directions opposées afin que l'instrument orientable (20, 220) prenne une forme en S lorsqu'il est actionné.

7. Système chirurgical (400, 460) selon l'une quelconque des revendications 1 à 5, dans lequel les positions angulaires des première et deuxième fentes (26, 236, 28, 248) sont ajustées progressivement autour de l'axe central de sorte que le segment de flexion (40, 222) se plie hors plan de manière hélicoïdale lorsqu'il est actionné.

8. Système chirurgical (400, 460) selon l'une quelconque des revendications 2 à 7, dans lequel les première et deuxième fentes (26, 236, 28, 246) comprennent des dispositifs de contact qui limitent le mouvement de flexion du segment de flexion (40, 222) dans des directions de flexion opposées,
éventuellement dans lequel chaque dispositif de contact comprend un élément de forme convexe faisant saillie d'un élément de poutre latérale positionné dans un récepteur concave de forme correspondante sur un élément de poutre latérale adjacent (54a) ; ou,
dans lequel chaque dispositif de contact présente une configuration en queue d'aronde, dans lequel l'élément de forme convexe comprend une goupille en queue d'aronde et le récepteur concave comprend une queue en queue d'aronde.

9. Système chirurgical (400, 460) selon l'une quelconque des revendications 1 à 8, dans lequel l'instrument orientable (20, 220) comprend une première tige d'insertion (320) s'étendant de manière proximale à partir de la première poutre serpentine (30, 234) et une deuxième tige d'insertion (320) s'étendant de manière proximale à partir de la deuxième poutre serpentine (32, 244), les première et deuxième tiges d'insertion (320) étant configurées pour être élastiques en flexion afin de leur permettre de s'adapter à un chemin tortueux, élastiques de manière omnidirectionnelle afin qu'elles présentent la même rigidité à la flexion, quelle que soit la direction de la charge, avec une rigidité axiale et une rigidité en torsion suffisantes pour transmettre suffisamment les forces et les couples des extrémités proximales (310) des tiges d'insertion (320) au segment de flexion distal (40, 222) de l'instrument orientable (20, 220).

10. Système chirurgical (400, 460) selon la revendication 9, dans lequel :
la première tige d'insertion (320) comprend un tube flexible en polymère (332) positionné à côté et relié au premier tube (22, 230), une couche de renfort en fil tressé (352) recouvrant ou intégrée au tube flexible (332), et une gaine en polymère (354) qui recouvre le fil tressé (352) et contribue à fixer le fil tressé (352) sur le tube flexible (332) ; et
la deuxième tige d'insertion (320) comprend un tube flexible polymère (332) positionné à côté et connecté au deuxième tube (24, 240), une couche de renfort en fil tressé (352) recouvrant ou intégrée dans le tube flexible (332), et une gaine en polymère (354) qui recouvre le fil tressé (352) et contribue à fixer le fil tressé (352) sur le tube flexible (332),
éventuellement, dans lequel la densité de tressage du fil tressé (352) est modifiée sur la longueur du au moins une des première et deuxième tiges d'insertion (320) afin d'ajuster la flexibilité, la rigidité axiale et la rigidité à la torsion de la tige d'insertion (320) sur sa longueur ; et,
en outre, en option, dans lequel les tubes flexibles (332) sont fixés à leurs deuxièmes tubes respectifs (24, 240) par l'un des procédés suivants : en intégrant le tube (24, 240) dans le matériau du tube flexible (332), en reliant le tube flexible (332) au tube extérieur (354) avec un adhésif, ou en formant le tube flexible (332) comme une fine gaine de matériau collée au tube extérieur (354).

11. Système chirurgical (400, 460) selon la revendication 9, dans lequel les première et deuxième tiges d'insertion (320) comprennent des portions respectives des premier et deuxième tubes (22, 230, 24, 240) s'étendant de manière proximale de leurs poutres serpentines respectives (30, 234, 32, 244) et comprenant une pluralité de fentes (26, 236, 28, 246) dans leurs parois latérales respectives, les fentes (26, 236, 28, 246) s'étendant perpendiculairement à l'axe central des premier et deuxième tubes (22, 230, 24, 240) et étant agencées en rangées s'étendant radialement autour de l'axe central et étant configurées de sorte que les première et deuxième tiges d'insertion (320) soient élastiques en flexion pour leur permettre d'adopter un trajet tortueux, élastiques dans toutes les directions afin qu'elles présentent la même rigidité à la flexion, quelle que soit la direction de la charge, et rigides axialement et en torsion afin qu'elles transmettent des forces et des couples des extrémités proximales des tiges d'insertion (320) au segment de flexion distal de l'instrument orientable (20, 220),
éventuellement dans lequel le pas des fentes (26, 236, 28, 246) agencées le long des première et deuxième tiges d'insertion (320) est modifié sur les longueurs des première et deuxième tiges d'insertion (320) pour ajuster la flexibilité, la rigidité axiale et la rigidité à la torsion des première et deuxième tiges d'insertion (320) sur leurs longueurs.

12. Système chirurgical (400, 460) selon la revendication 2, dans lequel l'instrument orientable (20, 220) comprend en outre une gaine en polymère (354) qui recouvre le segment de flexion (40, 222) et empêche les tissus de pénétrer dans les fentes (26, 236, 28, 246) ; ou,
dans lequel les fentes (26, 236, 28, 246) sont configurées pour être si étroites que les tissus ne peuvent pas entrer dans les fentes (26, 236, 28, 246).

13. Système chirurgical (400, 460) selon l'une quelconque des revendications précédentes, dans lequel l'instrument orientable (20, 220) comprend un instrument orientable composé comprenant un premier et un deuxième instrument orientable (410, 420), dans lequel le deuxième instrument orientable (420) est configuré pour s'étendre à travers une lumière interne du premier instrument orientable (410).

14. Système chirurgical (400, 460) selon l'une quelconque des revendications précédentes, comprenant en outre un dispositif d'administration, configuré pour être inséré et manipulé afin d'être positionné sur un site chirurgical, dans lequel l'instrument orientable (20, 220) est configuré pour être avancé à travers le dispositif d'introduction et pour faire en sorte que le segment de flexion (40, 222) soit positionné à l'extérieur du dispositif d'introduction, le reste de l'instrument orientable (20, 220) étant positionné à l'intérieur et soutenu par le dispositif d'administration,
éventuellement, dans lequel le dispositif d'administration comprend un endoscope, un endoscope flexible et un autre instrument orientable.

15. Système chirurgical (400, 460) selon la revendication 14, dans lequel le dispositif d'administration est configuré de manière à ce que l'instrument orientable (20, 220), supporté par le dispositif d'administration avec le segment de flexion (40, 222) positionné à l'extérieur du dispositif d'administration, puisse supporter un instrument chirurgical s'étendant à travers la lumière interne de la structure tubulaire imbriquée pour la manipulation des tissus sur le site chirurgical.
